# EUROPEAN PATENT APPLICATION

(11) **EP 4 403 571 A1**
(43) Date of publication of application: **24.07.2024**
(21) Application number: 22827670.5
(22) Date of filing: 23.06.2022
(51) Int. Cl.: C07K 16/28, C12N 15/13, C12N 15/63, C12N 5/10, A61K 39/395, A61P 35/00, G01N 33/53

(54) **HUMAN EPIDERMAL GROWTH FACTOR RECEPTOR BINDING MOLECULE AND USE THEREOF**

(30) Priority: 24.06.2021 CN 202110705939
(71) Applicant: Zhejiang Nanomab Technology Center Co. Ltd., Changle Township Shengzhou Shaoxing City Zhejiang Province, 312467 (CN)
(72) Inventor: LI, Jiaguo, Shanghai 201805 (CN); LIU, Xiangzhen, Shanghai 201805 (CN); ZHU, Weimin, Danville, California 94506 (US); SUN, Yan, Shanghai 201805 (CN); DING, Na, Shanghai 201805 (CN); QIAN, Qijun, Shanghai 201805 (CN)
(74) Representative: van Dam, Vincent
(86) International application number: PCT/CN2022/100888
(87) International publication number: WO 2022/268178

(57) **Abstract**

Provided in the present invention are a human epidermal growth factor receptor binding molecule and the use thereof. The human epidermal growth factor receptor binding molecule of the present invention contains a single domain antibody, which is against the human epidermal growth factor receptor. A complementary determining region (CDR) of the single domain antibody comprises CDR1, as represented by SEQ ID NO: 1, CDR2, as represented by SEQ ID NO: 2, and CDR3, as represented by SEQ ID NO: 3. Also provided in the present invention are a polynucleotide encoding the binding molecule, a nucleic acid construct containing same, and a corresponding phage, host cell, pharmaceutical composition, production method and non-diagnostic method, and the corresponding use.

## Description

### Technical Field

The invention relates to the biomedical or biopharmaceutical technology field, more specifically to a human epidermal growth factor receptor (EGFR) - binding molecule and its application.

### Background

Human epidermal growth factor receptor (EGFR, ErbB-1 or HER1) is a member of the epidermal growth factor receptor (HER) family. This family includes HER1 (ErbB1, EGFR), HER2 (ErbB2, neu), HER3 (ErbB3) and HER4 (ErbB4). EGFR is expressed on the surface of normal epithelial cells. There is high or abnormal expression of EGFR in many solid tumor cells, including head and neck cancer, breast cancer, bladder cancer, ovarian cancer, kidney cancer, colon cancer and non-small cell lung cancer, especially lung cancer. The EGFR mutation rate in Asian lung cancer population can reach 50% (seshacharyulu P et al., expert opin ther targets, 2012; 16: 15-31). EGFR is a popular target for tumor targeted therapy.

EGFR is located on the cell membrane surface and is activated by binding to ligands, including EGF and TGF α. After activation, EGFR is converted from monomers to dimers, although there is also evidence that dimers also exist before activation. EGFR may also aggregate with other members of the ErbB receptor family to activate. EGFR dimerization can activate its kinase pathways located in cells, including Y992, Y1045, Y1068, Y1148 and Y1173 activation sites. Autophosphorylation can guide downstream phosphorylation, including MAPK, Akt and JNK pathways. EGFR is related to the inhibition of tumor cell proliferation, angiogenesis, tumor invasion, metastasis and apoptosis. There are mainly two signal transduction pathways downstream of EGFR: one is Ras/Raf/MEK/ERK-MAPK pathway, and the other is PI3K/Akt/mTOR pathway (R roskoski Jr. pharmaceutical research, 2014:79:34-74.).

At present, there are four EGFR mAbs approved for marketing worldwide, namely cetuximab, panitumumab, anti cetuximab and nimotuzumab. It is mainly used to treat rectal cancer and head and neck cancer. Studies have shown that EGFR extracellular segment is very prone to mutation and drug resistance. However, the binding epitope of nano antibody is much smaller than that of scFv antibody, and it is less affected by mutations, which can improve the effective anti-tumor activity of EGFR to a certain extent. Moreover, nanoantibodies have the natural advantages of high stability, strong penetration and wide binding epitopes (muyldermans S. Annu Rev biochem. 2013; 82:775-97.). There is little research on nanoantibodies targeting the membrane proximal end of EGFR. It has become an urgent problem to develop a new type of anti human epidermal growth factor receptor nano antibody, which has good specificity, blocking activity, clinical efficacy, simple production, low cost, and reduce the drug burden.

### Summary

The invention aims to provide a novel anti-EGFR binding molecule and use thereof.

The first aspect of the invention provides a human epidermal growth factor receptor binding molecule, which comprises an anti-EGFR single domain antibody, wherein the complementarity determining region (CDR) of the single domain antibody comprises CDR1, CDR2 and CDR3, wherein CDR1 comprises the sequence shown in SEQ ID NO: 1, CDR2 comprises the sequence shown in SEQ ID NO: 2, and CDR3 comprises the sequence shown in SEQ ID NO: 3.

In one or more embodiments, SEQ ID NO: 1 is GX₁X₂X₃X₄X₅X₆X₇X₈, wherein X₁ is G, X₂ is S, F, G, L or R, X₃ is G, T, V, P, A, S or I, X₄ is F, L or D, X₅ is T, S, E, D, L or I, X₆ is I, D , SorT, X₇ is Q, Y, H or F, and X₈ is A or T.

In one or more embodiments, SEQ ID NO: 1 is GX₁X₂X₃X₄X₅X₆X₇X₈, wherein X₁ is G, X₂ is F or L, X₃ is T, P, or A, X₄ is F or L, X₅ is T, S, E, D, L or I, X₆ is D or T, X₇ is Y, and X₈ is A or T.

In one or more embodiments, CDR1 comprises the sequence shown in any one of SEQ ID NOs: 4-12, preferably, CDR1 comprises the sequence shown in any one of SEQ ID NOs:5,7, 8, 9 or 11.

In one or more embodiments, SEQ ID NO: 2 is X₁X₂X₃X₄X₅X₆X₇X₈, wherein X₁ is I, L or V, X₂ is H, T, F, A or S, X₃ is Q, G, S, P, T, I or N, X₄ is G, T, D, A, S or Y, X₅ is G, H ,E or N, X₆ is S, E, K, D, G or A, X₇ is T, H, I, K, S or null, and X₈ is S, T, I, P or null.

In one or more embodiments, SEQ ID NO: 2 is X₁X₂X₃X₄X₅X₆X₇X₈, wherein X₁ is I or L, X₂ is T, F, A or S, X₃ is G, S, P, T or I, X₄ is T, D, A, or S, X₅ is G or E, X₆ is E, K, D or G, X₇ is T, H, I, K, S or null, and X₈ is S, T, I or P.

In one or more embodiments, In one or more embodiments, CDR2 comprises the sequence shown in any one of SEQ ID NOs: 13-21, preferably, CDR2 comprises the sequence shown in any one of SEQ ID NOs: 14, 16,17,18, or 20.

In one or more embodiments, SEQ ID NO:3 is X₁X₂X₃X₄X₅X₆X₇X₈X₉X₁₀X₁₁X₁₂X₁₃X₁₄X₁₅X₁₆X₁₇X₁₈X₁₉X₂₀X₂₁ X₂₂X₂₃X₂₄X₂₅, wherein X₁ is N, S, A, K or H, X₂ is V, I, R, K, A or L, X₃ is V, Y, S, D, L, T or N, X₄ is P, H, L, V, S, K or Y, X₅ is P, Y, S, R, T or A, X₆ is L, P, T, F, A, S or D, X₇ is R, P, F, S, A, I or D, X₈ is V, D, G, A or Y, X₉ is Y, N, T, R, F or null, X₁₀ is P, A, W, E or null, X₁₁ is S, H, L, Y or null, X₁₂ is F, D, N, L or null, X₁₃ is Y, V or null, X₁₄ is I, G or null, X₁₅ is G or null, X₁₆ is Y, A or null, X₁₇ is G or null, X₁₈ is G or null, X₁₉ is G or null , X₂₀ is E or null, X₂₁ is V or null, X₂₂ is R or null, X₂₃ is Y or null, X₂₄ is E or null, X₂₅ is Y or null.

In one or more embodiments, SEQ ID NO:3 is X₁X₂X₃X₄X₅X₆X₇X₈X₉X₁₀X₁₁X₁₂X₁₃X₁₄X₁₅X₁₆X₁₇X₁₈X₁₉X₂₀X₂₁ X₂₂X₂₃X₂₄X₂₅, wherein X₁ is N, S, A or K, X₂ is I, K or A, X₃ is Y, D, L or T, X₄ is H, L, V, S or K, X₅ is P, S, R or T, X₆ is P, F, A, S or D, X₇ is R, P, F, S or I, X₈ is V, D, G or A, X₉ is Y, N, T, R or F, X₁₀ is P, A, W or null, X₁₁ is S, H, L or null, X₁₂ is F, D, N, L or null, X₁₃ is Y, V or null, X₁₄ is I, G or null, X₁₅ is G or null, X₁₆ is Y, A or null, X₁₇ is G or null, X₁₈ is G or null, X₁₉ is G or null, X₂₀ is E or null, X₂₁ is V or null, X₂₂ is R or null, X₂₃ is Y or null, X₂₄ is E or null, X₂₅ is Y or null.

In one or more embodiments, CDR3 comprises the sequence shown in any one of SEQ ID NOs: 22-30, preferably, CDR3 comprises the sequence shown in any one of SEQ ID NOs: 23, 25, 26, 27, or 29.

In one or more embodiments, the anti human epidermal growth factor receptor single domain antibody is a specific single domain antibody to the membrane proximal region III or IV of the human epidermal growth factor receptor.

In one or more embodiments, CDR1 comprises the sequence shown in any one of SEQ ID NOs: 4-12, CDR2 comprises the sequence shown in any one of SEQ ID NOs: 13-21, and CDR3 comprises the sequence shown in any one of SEQ ID NOs:22-30.

In one or more embodiments, the single domain antibody comprises CDR1, CDR2, and CDR3 shown in any one of the following groups a1 to a9:

| Group | CDR1 | CDR2 | CDR3 |
|---|---|---|---|
| a1 | 4 | 13 | 22 |
| a2 | 5 | 14 | 23 |
| a3 | 6 | 15 | 24 |
| a4 | 7 | 16 | 25 |
| a5 | 8 | 17 | 26 |
| a6 | 9 | 18 | 27 |
| a7 | 10 | 19 | 28 |
| a8 | 11 | 20 | 29 |
| a9 | 12 | 21 | 30 |

In one or more embodiments, FR1 region of the single domain antibody VHH is the FR1 region of any VHH selected from SEQ ID NOs: 31-82, FR2 region of VHH is the FR2 region of any VHH selected from SEQ ID NOs: 31-82, FR3 region of VHH is the FR3 region of any VHH selected from SEQ ID NOs: 31-82, and FR4 region of VHH is the FR4 region of any VHH selected from SEQ ID NOs: 31-82.

In one or more embodiments, the FR region of the single domain antibody is the FR region of any VHH selected from SEQ ID NOs: 31-82.

In one or more embodiments, the single domain antibody VHH is as shown in any one of SEQ ID NOs: 31-82, preferably, the single domain antibody VHH is as shown in any one of SEQ ID NOs:40-82.

In one or more embodiments, the human epidermal growth factor receptor binding molecule is a monovalent or multivalent single domain antibody, a multispecific single domain antibody, a heavy chain antibody or an antigen binding fragment thereof, an antibody or an antigen binding fragment thereof comprising one, two or more of the anti-EGFR single domain antibodies described herein.

In one or more embodiments, the multivalent single domain antibody or multispecific single domain antibody connects a plurality of single domain antibodies through a linker. The linker consists of 1-15 amino acids selected from G and S.

In one or more embodiments, the antigen binding fragment of the heavy chain antibody is a single chain heavy chain antibody.

In one or more embodiments, the heavy chain antibody is a camelid heavy chain antibody or a shark heavy chain antibody.

In one or more embodiments, the heavy chain antibody further comprises a heavy chain constant region.

In one or more embodiments, the heavy chain constant region is a constant region of camelid heavy chain antibody, comprising CH2 and CH3. In one or more embodiments, the CH2 and CH3 are CH2 and CH3 of human IgG Fc, such as CH2 and CH3 of IgG1 or IgG4. Preferably, the heavy chain constant region is CH2 and CH3 of IgG4, and its amino acid sequence is shown in SEQ ID NO: 83.

In one or more embodiments, the heavy chain constant region is a constant region of the shark heavy chain antibody, comprising CH1, CH2, CH3, CH4, and CH5.

In one or more embodiments, the antibody is an antibody comprising the anti-EGFR single domain antibody as the heavy chain variable domain.

In one or more embodiments, the antibody further comprises a light chain variable domain, a heavy chain constant domain, and a light chain constant domain.

In one or more embodiments, the antigen binding fragment of the antibody is selected from Fab, F(ab')2, Fv, scFv.

In one or more embodiments, the binding molecule described in any embodiment of the present description is a chimeric antibody or a fully human antibody; preferably, a fully human antibody.

The description also provides a polynucleotide, comprising a sequence selected from:
(1) a coding sequence of the single domain antibody or the antibody or the antigen binding fragment thereof according to any embodiment herein;
(2) a complementary sequence of (1);
(3) a 5-50bp fragment of any sequence of (1) or (2).

In one or more embodiments, the fragment is primer.

The description also provides a nucleic acid construct comprising the polynucleotide described herein.

In one or more embodiments, the nucleic acid construct is a recombinant vector or expression vector.

The description also provides a phage comprising the human epidermal growth factor receptor binding molecule according to any embodiment herein.

In one or more embodiments, the human epidermal growth factor receptor binding molecule is displayed on the surface of the phage.

The description also provides a host cell selected from:
(1) the host cell expressing the human epidermal growth factor receptor binding molecule according to any embodiment herein;
(2) the host cell comprising a polynucleotide described herein; and/or
(3) the host cell comprising a nucleic acid construct described herein.

The description also provides a method for producing a human epidermal growth factor receptor binding molecule, comprising culturing the host cells described herein under conditions suitable for producing human epidermal growth factor receptor binding molecule (such as monovalent or multivalent single domain antibodies, multispecific single domain antibodies, heavy chain antibodies, antibodies or antigen binding fragments thereof), and optionally purifying the human epidermal growth factor receptor binding molecule from culture.

The description also provides a pharmaceutical composition, comprising the human epidermal growth factor receptor binding molecule, polynucleotide, nucleic acid construct, phage or host cell according to any embodiment herein, and a pharmaceutically acceptable excipient.

In one or more embodiments, the pharmaceutical composition is used for treating cancer.

In one or more embodiments, the cancer is the human epidermal growth factor receptor related cancer. Preferably, the cancer is selected from the group consisting of: head and neck cancer, breast cancer, bladder cancer, ovarian cancer, renal carcinoma, colon cancer, non-small cell lung cancer, and the like.

The description also provides use of the human epidermal growth factor receptor binding molecule according to any embodiment herein in the preparation of a medicament for the prevention or treatment of a cancer.

In one or more embodiments, the cancer is a human epidermal growth factor receptor related cancer. Preferably, the cancer is selected from the group consisting of: head and neck cancer, breast cancer, bladder cancer, ovarian cancer, renal carcinoma, colon cancer, non-small cell lung cancer, and the like.

The description also provides a method for treating or preventing a cancer, comprising administrating a patient in need thereof an effective amount of a human epidermal growth factor receptor binding molecule according to any embodiment of the description, or a pharmaceutical compisiton comprising a human epidermal growth factor receptor binding molecule according to any embodiment of the description.

In one or more embodiments, the cancer is a human epidermal growth factor receptor related cancer. Preferably, the cancer is selected from the group consisting of: head and neck cancer, breast cancer, bladder cancer, ovarian cancer, renal carcinoma, colon cancer, non-small cell lung cancer, and the like.

The description also provides a kit for detecting human epidermal growth factor receptor, for use in evaluating the therapeutic effect of a medicament or diagnosing cancer. The kit comprises a human epidermal growth factor receptor binding molecule, polynucleotide, nucleic acid construct, phage or host cell according to any embodiment of the description.

In one or more embodiments, the kit further comprises a reagent for detecting the binding of human epidermal growth factor receptor to a single domain antibody, an antibody, or an antigen binding fragment thereof. For example, the bound reagent is detected by the enzyme-linked immunosorbent assay.

In one or more embodiments, the detection reagent for binding is a detectable marker, such as biotin, that can be linked to a human epidermal growth factor receptor binding molecule. The detectable marker is connected to the human epidermal growth factor receptor binding molecule or present in the kit separately.

The description also provides a non diagnostic method for detecting the presence of human epidermal growth factor receptor in a sample. The method comprises: incubating a human epidermal growth factor receptor binding molecule according to any embodiment herein with the sample, and detecting the binding of human epidermal growth factor receptor to a single domain antibody, antibody, or antigen binding fragment thereof, thereby determining the presence of human epidermal growth factor receptor in the sample. The detection is an enzyme-linked immunosorbent assay.

The description also provides use of a human epidermal growth factor receptor binding molecule according to any embodiment herein in the preparation of a kit for detecting human epidermal growth factor receptor in a sample, evaluating the therapeutic effect of a medicament or diagnosing a cancer.

### Detailed Description

Figure 1 shows A. B is the titer detection diagram of Alpaca antiserum against EGFR his protein and EGFR-III region protein respectively.
In Figure 2, A-D shows the binding of VHH-IgG4 antibody to the full-length EGFR extracellular domain protein, I+II domain protein, III domain protein, and IV domain protein, respectively. Except E009 antibody, which binds to I+II region, other antibodies bind to III and IV regions of EGFR extracellular segment.
Figure 3 shows VHH-IgG4 antibody competed with EGF for binding to EGFR antigen by ELISA.
Figure 4 shows the binding detection results of VHH-IgG4 antibody and 293T stably transformed cell line overexpressing EGFR III region protein.
Figure 5 shows the binding test results of VHH-IgG4 antibody and SKOV3 tumor cell line.
Figure 6 shows the binding test results of VHH-IgG4 antibody and Aspc-1 tumor cell line.
In Figure 7, A-C are the results of membrane protein array screening (MPA) of antibodies E002, E005 and E008 respectively.In the figure, the unit of IC50 and EC50 is nM.

### Detailed Description

After extensive and in-depth research and extensive screening, the inventor found a class of human epidermal growth factor receptor binding molecules containing anti human epidermal growth factor receptor single domain antibodies. The human epidermal growth factor receptor binding molecule of the invention can bind to the membrane proximal region III or IV of EGFR antigen with high specificity, and high affinity , resulting in high biological activity, and has low immunogenicity, has stable structure, and has good druggability. The single domain antibody of the invention is simple to generate.

### Antibody

"Human epidermal growth factor receptor binding molecule" or "EGFR binding molecule" as used herein is a protein that specifically binds Human epidermal growth factor receptor, including but not limited to antibodies, antigen binding fragments of antibodies, heavy chain antibodies, nano antibodies, micro antibodies, affibodies, target binding regions of receptors, cell adhesion molecules, ligands, enzymes, cytokines, and chemokines.

"Human epidermal growth factor receptor membrane proximal region III" (EGFR Region III) refers to the region of amino acids 311-480 of human epidermal growth factor receptor (uniprot, P00533-1, C311-L480).

"Human epidermal growth factor receptor proximal membrane region IV" (EGFR Region IV) refers to the region of amino acids 481-645 of human epidermal growth factor receptor (uniprot, P00533-1, F481-S645).

Term "antibody" as used herein includes monoclonal antibodies (including full-length antibodies with immunoglobulin Fc region), antibody compositions with polyepitopic specificity, multi-specific antibodies (e.g., bispecific antibodies), diabodies and single chain molecules, and antibody fragments, especially antigen binding fragments, (e.g., Fab, F(ab')2, and FV). The term "immunoglobulin" (Ig) is used interchangeably with "antibody" herein.

The basic 4-chain antibody unit is a heterotetrameric glycoprotein composed of two identical light chains (L) and two identical heavy chains (H). IgM antibody consists of 5 basic heterotetramer units and another polypeptide called J chain, which contains 10 antigen binding sites; IgA antibody contains 2-5 basic 4 chain units, which can polymerize with J chain to form a multivalent assemblages. In the case of IgGs, the 4-chain unit is typically about 150,000 daltons. Each light chain is connected to a heavy chain through a covalent disulfide bond, while the two heavy chains are connected to each other through one or more disulfide bonds, and the number of disulfide bonds depends on the isotype of the heavy chain. Each heavy and light chain also has an interchain disulfide bridge with regular spacing. Each heavy chain has a variable domain (VH) at the N-terminus, followed by three constant domains (for each α and γ chain, CH1, CH2, and CH3) and four constant domains (for µ and ε isoforms, CH1, CH2, CH3, and CH4) and the hinge region (Hinge) between the CH1 domain and the CH2 domain. Each light chain has a variable domain (VL) at the N-terminus, followed by a constant domain (CL) at the other end. VL is aligned with VH, while CL is aligned with the first constant domain (CH1) of the heavy chain. Specific amino acid residues are thought to form an interface between light and heavy chain variable domains. The paired VH and VL together form an antigen binding site. For the structures and properties of different classes of antibodies, see, for example, Basic and Clinical Immunology, 8th Edition, Daniel P. Sties, Abba I. Terr, and Tristram G. Parsolw, ed, Appleton & Lange, Norwalk, CT, 1994, page 71, and Chapter 6. The L chain from any vertebrate species can be assigned to one of two clearly distinct types, called kappa and lambda, based on the amino acid sequences of their constant domains. Depending on the amino acid sequence of the constant domain of their heavy chains (CH), immunoglobulins can be assigned to different classes or isotypes. There are five classes of immunoglobulins: IgA, IgD, IgE, IgG and IgM, having heavy chains designated α, δ, ε, γ and µ, respectively. The γ and α classes are further divided into subclasses on the basis of relatively minor differences in the CH sequence and function, e.g., humans express the following subclasses: IgG1, IgG2A, IgG2B, IgG3, IgG4, IgA1 and IgA2.

"Heavy chain antibody" described herein is an antibody derived from camelidae or sharks. Compared with the above 4-chain antibody, the heavy chain antibody lacks light chain and heavy chain constant region 1 (CH1), and only contains two heavy chains composed of variable region (VHH) and other constant regions, wherein the variable region is connected to the constant region through a hinge region like structure. Each heavy chain of camelid heavy chain antibody contains one variable region (VHH) and two constant regions (CH2 and CH3), and each heavy chain of shark heavy chain antibody contains one variable region and five constant regions (CH1-CH5). Antigen binding fragments of heavy chain antibodies include VHH and single chain heavy chain antibodies. Heavy chain antibodies may have CH2 and CH3 of human IgG Fc by fusing with the constant region of human IgG Fc.

As used herein, the terms "single domain antibody", "anti-EGFR single domain antibody", "heavy chain variable region domain of heavy chain antibody", "VHH" and "nanobody" can be used interchangeably, and all refer to single domain antibodies that specifically recognize and bind to human epidermal growth factor receptor. Single domain antibodies are variable regions of heavy chain antibodies. Typically, single domain antibodies contain three CDRs and four FRs. Preferably, the single domain antibody of the description has CDR1 shown in SEQ ID NO: 1, CDR2 shown in SEQ ID NO: 2, and CDR3 shown in SEQ ID NO: 3. Single domain antibodies are the smallest functional antigen binding fragments. Generally, after obtaining an antibody which naturally lacks light chain and heavy chain constant region 1 (CH1), the variable region of the heavy chain of the antibody is cloned to construct a single domain antibody consisting of only one heavy chain variable region.

Binding molecules comprising two or more single domain antibodies are multivalent single domain antibodies; and binding molecules comprising two or more single domain antibodies with different specificities are multispecific single domain antibodies. Multivalent single domain antibodies or multispecific single domain antibodies are connected to multiple single domain antibodies through linkers. The linker usually consists of 1-15 amino acids selected from G and S.

The terms "heavy chain antibody" and "antibody" herein are intended to distinguish different composition forms of antibodies. Due to the similarity of their structures, the following descriptions on structures of antibodies except for light chains also apply to heavy chain antibodies.

The "variable region" or "variable domain" of an antibody refers to the amino-terminal domains of the heavy or light chain of the antibody. The variable domains of the heavy chain and light chain may be referred to as "VH" and "VL", respectively. These domains are generally the most variable parts of the antibody (relative to other antibodies of the same class) and contain the antigen binding sites.

The term "variable" refers to the fact that certain segments of the variable domains differ extensively in sequence among antibodies. The V domain mediates antigen binding and defines the specificity of a particular antibody for its particular antigen. However, the variability is not evenly distributed across the entire span of the variable domains. Instead, it is concentrated in three segments called hypervariable regions (HVRs) both in the light-chain and the heavy chain variable domains, namely HCDR1, HCDR2 and HCDR3 of heavy chain variable region (CDR1, CDR2 and CDR3 in heavy chain antibodies for short) and LCDR1, LCDR2 and LCDR3 of light chain variable region. The more highly conserved portions of variable domains are called the framework regions (FR). The variable domains of native heavy and light chains each comprise four FR regions (FR1, FR2, FR3, and FR4), largely adopting a beta-sheet configuration, connected by three HVRs, which form loops connecting, and in some cases forming part of, the beta-sheet structure. The HVRs in each chain are held together in close proximity by the FR regions and, with the HVRs from the other chain, contribute to the formation of the antigen binding site of antibodies. Generally, the structure of the light chain variable region is FR1-LCDR1-FR2-LCDR2-FR3-LCDR3-FR4, and the structure of the heavy chain variable region is FR1-HCDR1-FR2-HCDR2-FR3-HCDR3-FR4. The constant domains are not involved directly in the binding of antibody to an antigen, but exhibit various effector functions, such as participation of the antibody in antibody-dependent cellular toxicity.

An "Fc region" (fragment crystallizable region) or "Fc domain" or "Fc" refers to the C-terminal region of the heavy chain of an antibody that mediates the binding of the immunoglobulin to host tissues or factors, including binding to Fc receptors located on various cells of the immune system (e.g., effector cells) or to the first component (Clq) of the classical complement system. In IgG, IgA and IgD antibody isotypes, the Fc region is composed of two identical protein fragments, derived from CH2 and CH3 constant domains of the antibody's two heavy chains; IgM and IgE Fc regions contain three heavy chain constant domains (CH domains 2-4) in each polypeptide chain. Although the boundaries of the Fc region of an immunoglobulin heavy chain might vary, the human IgG heavy chain Fc region is usually defined to stretch from an amino acid residue at position C226 or P230 to the carboxy-terminus of the heavy chain, wherein the numbering is according to the EU index as in Kabat. As used herein, the Fc region may be a native sequence Fc or a variant Fc.

An "antibody fragment" comprises a portion of an intact antibody, preferably the antigen binding and/or the variable region of the intact antibody. The antibody fragment is preferably an antigen binding fragment of the antibody. Examples of antibody fragments include Fab, Fab', F(ab')2 and Fv fragments; diabodies; linear antibodies; single-chain antibody molecules, scFv-Fc fragment; multispecific antibodies formed from antibody fragments; and any fragment that should be able to increase the half-life by chemical modification or by incorporation into liposomes. Papain digestion of antibodies produced two identical antigen-binding fragments, called "Fab" fragments, and a residual "Fc" fragment, a designation reflecting the ability to crystallize readily. The Fab fragment consists of an entire L chain along with the variable region domain of the H chain (VH), and the first constant domain of one heavy chain (CH1). Each Fab fragment is monovalent with respect to antigen binding, i.e., it has a single antigen-binding site. Pepsin treatment of an antibody yields a single large F(ab')2 fragment which roughly corresponds to two disulfide linked Fab fragments having different antigen-binding activity and is still capable of cross-linking antigen. Fab' fragments differ from Fab fragments by having a few additional residues at the carboxy terminus of the CH1 domain including one or more cysteines from the antibody hinge region. F(ab')2 antibody fragments originally were produced as pairs of Fab' fragments which have hinge cysteines between them. Other chemical couplings of antibody fragments are also known. The Fc fragment comprises the carboxy-terminal portions of both H chains held together by disulfides. The effector functionfunctions of an antibody isantibodies are determined by the sequencesequences in the Fc region, the region which is also recognized by Fc receptors (FcRs) found on somecertain types of cells.

"Fv" is the minimum antibody fragment which contains a complete antigen-recognition and -binding site. This fragment consists of a dimer of one heavy- and one light-chain variable region domain in tight, non-covalent association. From the folding of these two domains emanate six hypervariable loops (3 loops each from the H and L chain) that contribute the amino acid residues for antigen binding and confer antigen binding specificity to the antibody. However, even a single variable domain (or half of an Fv comprising only three HVRs specific for an antigen) has the ability to recognize and bind antigen, although at a lower affinity than the entire binding site. "Single-chain Fv " also abbreviated as "sFv " or "scFv " are antibody fragments that comprise the VH and VL antibody domains connected into a single polypeptide chain. Preferably, the sFv polypeptide further comprises a polypeptide linker between the VH and VL domains which enables the sFv to form the desired structure for antigen binding.

The term "monoclonal antibody" as used herein refers to an antibody obtained from a population of substantially homogeneous antibodies, i.e., the individual antibodies comprising the population are identical except for possible naturally occurring mutations and/or post-translation modifications (e.g., isomerizations, amidations) that may be present in minor amounts. Monoclonal antibodies are highly specific, being directed against a single antigenic site. In contrast to polyclonal antibody preparations which typically include different antibodies directed against different determinants (epitopes), each monoclonal antibody is directed against a single determinant on the antigen In addition to their specificity, the monoclonal antibodies are advantageous in that they are synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including, for example, the hybridoma method, phage-display technologies, recombinant DNA methods, and technologies for producing human or humanlike antibodies in animals that have parts or all of the human immunoglobulin loci or genes encoding human immunoglobulin sequences, single-cell sequencing methods.

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is(are) identical with or homologous to corresponding sequences in antibodies derived from another species or belonging to another antibody class or subclass, as well as fragments of such antibodies, so long as they exhibit the desired biological activity.

"Humanized" forms of non-human (e.g., murine) antibodies are chimeric antibodies that contain minimal sequence derived from non-human immunoglobulin. Therefore, "humanized antibodies" generally refer to non-human antibodies that have had the variable-domain framework regions swapped for sequences found in human antibodies. Generally, in a humanized antibody, the entire antibody, except the CDRs, is encoded by a polynucleotide of human origin or is identical to such an antibody except within its CDRs. The CDRs, some or all of which are encoded by nucleic acids originating in a non-human organism, are grafted into the beta-sheet framework of a human antibody variable region to create an antibody, the specificity of which is determined by the engrafted CDRs. The creation of such antibodies are well known in the art, such as using mice with genetically engineered immune systems. In the description, antibodies, single domain antibodies, heavy chain antibodies, etc. all include humanized variants of the antibodies.

A "human antibody" is an antibody that possesses an amino-acid sequence corresponding to that of an antibody produced by a human and/or has been made using any of the techniques for making human antibodies as disclosed herein. This definition of a human antibody specifically excludes a humanized antibody comprising non-human antigen-binding residues. Human antibodies can be produced using various techniques known in the art, including phage-display libraries.

In some embodiments, the description further provides a single domain antibody, heavy chain antibody, antibody or antigen binding fragment thereof that binds to the same epitope of human epidermal growth factor receptor as any anti-EGFR single domain antibody of the description (For example, human epidermal growth factor receptor membrane proximal region III and IV), that is, a single domain antibody, heavy chain antibody, antibody or antigen binding fragment thereof that can cross-compete with any single domain antibody of the description for binding to human epidermal growth factor receptor.

In the present invention, the CDR1 of the anti-EGFR single domain antibody comprises the sequence shown in SEQ ID NO: 1, and SEQ ID NO: 1 is GX₁X₂X₃X₄X₅X₆X₇X₈, wherein X₁ is G, X₂ is S, F, G, L or R, X₃ is G, T, V, P, A, S or I, X₄ is F, L or D, X₅ is T, S, E, D, L or I, X₆ is I, D, SorT, X₇ is Q, Y, H or F, and X₈ is A or T. Preferably, X₁ is G, X₂ is F or L, X₃ is T, P, or A, X₄ is F or L, X₅ is T, S, E, D, L or I, X₆ is D or T, X₇ is Y, and X₈ is A or T.

In one or more embodiments, CDR1 comprises the sequence shown in any one of SEQ ID NOs: 4-12.

In the present invention, The CDR2 of the anti-EGFR single domain antibody comprises the sequence shown in SEQ ID NO: 2, SEQ ID NO: 2 is X₁X₂X₃X₄X₅X₆X₇X₈, wherein X₁ is I, L or V, X₂ is H, T, F, A or S, X₃ is Q, G, S, P, T, I or N, X₄ is G, T, D, A, S or Y, X₅ is G, H ,E or N, X₆ is S, E, K, D, G or A, X₇ is T, H, I, K, S or null, and X₈ is S, T, I, P or null. Preferably, X₁ is I or L, X₂ is T, F, A or S, X₃ is G, S, P, T or I, X₄ is T, D, A, or S, X₅ is G or E, X₆ is E, K, D or G, X₇ is T, H, I, K, S or null, and X₈ is S, T, I or P.

In one or more embodiments, CDR2 comprises the sequence shown in any one of SEQ ID NOs:13-21.

The CDR3 of the anti-EGFR single domain antibody comprises the sequence shown in SEQ ID NO: 3, SEQ ID NO: 3 is X₁X₂X₃X₄X₅X₆X₇X₈X₉X₁₀X₁₁X₁₂X₁₃X₁₄X₁₅X₁₆X₁₇X₁₈X₁₉X₂₀X₂₁X₂₂, wherein X₁ is N, S, A, K or H, X₂ is V, I, R, K, A or L, X₃ is V, Y, S, D, L, T or N, X₄ is P, H, L, V, S, K or Y, X₅ is P, Y, S, R, T or A, X₆ is L, P, T, F, A, S or D, X₇ is R, P, F, S, A, I or D, X₈ is V, D, G, A or Y, X₉ is Y, N, T, R, F or null, X₁₀ is P, A, W, E or null, X₁₁ is S, H, L, Y or null, X₁₂ is F, D, N, L or null, X₁₃ is Y, V or null, X₁₄ is I, G or null, X₁₅ is G or null , X₁₆ is Y, A or null, X₁₇ is G or null, X₁₈ is G or null, X₁₉ is G or null, X₂₀ is E or null, X₂₁ is V or null, X₂₂ is R or null, X₂₃ is Y or null, X₂₄ is E or null, X₂₅ is Y or null. Preferably, X₁ is N, S, A or K, X₂ is I, K or A, X₃ is Y, D, L or T, X₄ is H, L, V, S or K, X₅ is P, S, R or T, X₆ is P, F, A, S or D, X₇ is R, P, F, S or I, X₈ is V, D, G or A, X₉ is Y, N, T, R or F, X₁₀ is P, A, W or null, X₁₁ is S, H, L or null, X₁₂ is F, D, N, L or null, X₁₃ is Y, V or null, X₁₄ is I, G or null, X₁₅ is G or null, X₁₆ is Y, A or null, X₁₇ is G or null, X₁₈ is G or null, X₁₉ is G or null, X₂₀ is E or null, X₂₁ is V or null, X₂₂ is R or null, X₂₃ is Y or null, X₂₄ is E or null, X₂₅ is Y or null.

In one or more embodiments, CDR3 comprises the sequence shown in any one of SEQ ID NOs: 22-30.

In one or more embodiments, The CDR1 of the anti-EGFR single domain antibody comprises the sequence shown in any one of SEQ ID NOs:5,7, 8, 9 or 11. CDR2 comprises the sequence shown in any one of SEQ ID NOs: 14, 16,17,18, or 20. CDR3 comprises the sequence shown in any one of SEQ ID NOs: 23, 25, 26, 27, or 29.

In one or more embodiments, the single domain antibody comprises CDR1, CDR2, and CDR3 shown in any one of the following groups a1 to a9:

| Group | CDR1 | CDR2 | CDR3 |
|---|---|---|---|
| a1 | 4 | 13 | 22 |
| a2 | 5 | 14 | 23 |
| a3 | 6 | 15 | 24 |
| a4 | 7 | 16 | 25 |
| a5 | 8 | 17 | 26 |
| a6 | 9 | 18 | 27 |
| a7 | 10 | 19 | 28 |
| a8 | 11 | 20 | 29 |
| a9 | 12 | 21 | 30 |

Preferably, containing CDR1, CDR2, and CDR3 selected from any of the following groups: a2, a4, a5, a6, or a8.

In one or more embodiments, FR1 region of the single domain antibody VHH is the FR1 region of any VHH selected from SEQ ID NOs:31-82, FR2 region of VHH is the FR2 region of any VHH selected from SEQ ID NOs: 31-82, FR3 region of VHH is the FR3 region of any VHH selected from SEQ ID NOs: 31-82, and FR4 region of VHH is the FR4 region of any VHH selected from SEQ ID NOs: 31-82.

In a preferred embodiment, the FR region of the single domain antibody VHH of the invention is the FR region of any VHH selected from SEQ ID NOs: 31-82. More preferably, the CDR of such antibodies is selected from any of the aforementioned groups a1 to a9. In one or more embodiments, the single domain antibody VHH is as shown in any one of SEQ ID NOs:31-38, 40-46, 57-82.

The human epidermal growth factor receptor binding molecule described herein may be a monovalent or multivalent single domain antibody, a multispecific single domain antibody, a heavy chain antibody, or an antigen binding fragment thereof, an antibody, or an antigen binding fragment thereof, comprising one, two, or more anti-EGFR single domain antibodies described herein. The heavy chain antibody further comprises a heavy chain constant region, such as a constant region of camelid heavy chain antibody or shark heavy chain antibody. Preferably, the heavy chain constant region is shown in SEQ ID NO: 83.

The description also comprises the antibody derivatives and analogues. "Derivatives" and "analogues" refer to polypeptides that basically maintain the same biological function or activity of the antibody of the present description. The derivatives or analogues of the present description may be polypeptides formed from (i) a polypeptide with a substituent group in one or more amino acid residues, or (ii) a polypeptide formed from fusion of a mature polypeptide with another compound, such as a compound that extends the half-life of the polypeptide, such as polyethylene glycol, or (iii) a polypeptide formed by fusing an additional amino acid sequence to this polypeptide sequence (such as a leader sequence or a secretory sequence, or a sequence or prokaryotic sequence used for purifying this polypeptide, or a fusion protein formed with a 6His tag). According to the teaching herein, these derivatives and analogues belong to common sense known to those skilled in the art.

Without substantially affecting the activity of the antibody, those skilled in the art may change one or more (for example, 1, 2, 3, 4, 5, 6, 7, 8, 9 or 10 or more) amino acids to the sequence of the description to obtain the variant of the antibody or the functional fragment sequence thereof. These variants include (but are not limited to): deletion, insertion, and/or substitution of one or more (usually 1-50, preferably 1-30, more preferably 1-20, and most preferably 1-10) amino acids, and addition of one or more (usually less than 20, preferably less than 10, and more preferably less than 5) amino acids at the C-terminus and/or N-terminus. In this field, conservative substitution with amino acids with similar or similar properties usually does not change the function of the protein. For example, substituting with amino acids having similar properties may be performed in the FR and/or CDR regions of the variable region. Amino acid residues available for conservative substitution are well known in the art. Such substituted amino acid residues may or may not be encoded by a genetic code. For another example, adding one or more amino acids to the C-terminus and/or N-terminus usually does not change the function of the protein. They are all considered to be included in the scope of the present description.

The variant forms of the antibody described herein include: homologous sequence, conservative variant, allelic variant, natural mutant, induced mutant, protein encoded by DNA that can hybridize with the coding DNA of the antibody of the description under high or low strictness conditions, and polypeptide or protein obtained by using the antiserum of the antibody of the description.

In some embodiments, the sequence of the variant of the present description may have at least 95%, 96%, 97%, 98% or 99% identity with its source sequence. The sequence identity described in the description can be measured using sequence analysis software. For example, the computer program BLAST with default parameters, especially BLASTP or TBLASTN. The description also comprises those molecules with variable regions of antibody heavy chain with CDRs, if their CDRs have more than 90% homology (preferably more than 95%, more preeferably more than 98%) with the CDRs identified here.

The antibody of the description can be prepared by conventional methods in the art, such as hybridoma technology well known in the art. The heavy chain antibody of the description can be prepared by conventional methods in the art, such as phage display technology well known in the art. Alternatively, the antibodies or heavy chain antibodies of the present description may be expressed in other cell lines. Suitable mammalian host cells can be transformed with sequences encoding the antibodies of the present description. Transformation can be carried out using any known method, including, for example, packaging polynucleotides in viruses (or viral vectors) and transducing host cells with the viruses (or vectors). The transformation procedure used depends on the host to be transformed. Methods for introducing heterologous polynucleotides into mammalian cells are well known in the art, including dextran mediated transfection, calcium phosphate precipitation, Polybrene mediated transfection, protoplast fusion, electroporation, encapsulation of polynucleotides in liposomes and direct microinjection of DNA into the nucleus. Mammalian cell lines that can be used as hosts for expression are well known in the art, including but not limited to a variety of immortalized cell lines available from the American Typical Culture Collection (ATCC), including but not limited to Chinese hamster ovary (CHO) cells, HeLa cells, baby hamster kidney (BHK) cells, monkey kidney cells (COS), human hepatocellular carcinoma cells (e.g., HepG2), etc. Particularly preferred cell lines are selected by determining which cell lines have high expression levels and produce antibodies with substantial human epidermal growth factor receptor binding properties.

### Nucleic Acid

The description also provides polynucleotides encoding the above antibody or fragments thereof. Polynucleotides encoding heavy chain variable region, light chain variable region, heavy chain, light chain and CDRs are provided. The polynucleotide of the description can be in the form of DNAs or RNAs. DNAs include cDNAs, genomic DNAs, or synthetic DNAs. DNAs can be single stranded DNAs or double stranded DNAs. DNAs can be coding or noncoding strand DNAs.

As those skilled in the art will understand, due to the degeneracy of the genetic code, an extremely large number of nucleic acids can be prepared, all of which encode the antibody of the description or antigen binding fragment thereof. Therefore, when a specific amino acid sequence has been identified, those skilled in the art can simply modify the sequence of one or more codons without changing the amino acid sequence of the encoded protein to produce any number of different nucleic acids. Therefore, the present description also relates to polynucleotides that hybridize with the above polynucleotide sequences and have at least 50%, preferably at least 70%, more preferably at least 80% identity between the two sequences. The description particularly relates to polynucleotides that can hybridize with the polynucleotides of the description under strict conditions. In the present description, "strict conditions" refer to: (1) hybridization and elution at lower ionic strength and higher temperature, such as 0.2 × SSC, 0.1%SDS, 60 °C; or (2) addition of denaturants during hybridization, such as 50% (v/v) formamide, 0.1% calf serum/0.1% Ficoll, 42 °C, etc; or (3) hybridization that occurs only when the identity between two sequences is at least more than 90%, or preferably, more than 95%. Moreover, the polypeptides encoded by hybridizable polynucleotides have the same biological functions and activities as mature polypeptides.

The nucleotide full-length sequence of the antibody of the description or fragment thereof can usually be obtained by PCR amplification method, recombination method or artificial synthesis method. A feasible method is to synthesize relevant sequences by artificial synthesis, especially with short fragment length. Usually, fragments with very long sequence can be obtained by synthesizing several small fragments first and then connecting them. In addition, the coding sequence of the heavy chain and the expression tag (such as 6His) can also be fused together to form a fusion protein.

Once the relevant sequences are obtained, they can be obtained in large quantities by recombination. They are related sequences usually cloned into vectors, transferred into cells, and then isolated from the proliferated host cells by conventional methods. The biomolecules (nucleic acids, proteins, etc.) according to the present description include biomolecules in isolated form. At present, the DNA sequence encoding the protein (or fragment thereof, or derivative thereof) of the description can be obtained completely through chemical synthesis. This DNA sequence can then be introduced into various existing DNA molecules (or such as vectors) and cells known in the art. In addition, mutations can also be introduced into the protein sequence of the description through chemical synthesis.

Therefore, the present description also relates to nucleic acid constructs, such as expression vectors and recombinant vectors, comprising the above appropriate DNA sequence and the appropriate promoter or control sequence. These vectors can be used to transform appropriate host cells to enable them to express proteins. Vectors usually contain sequences for plasmid maintenance and for cloning and expressing exogenous nucleotide sequences. The sequences (collectively referred to as "flanking sequence" in some embodiments) generally comprises one or more of the following nucleotide sequences: promoter, one or more enhancer sequences, replication origin, transcription termination sequence, complete intronic sequence comprising donor and receptor splice sites, sequence encoding leader sequence for polypeptide secretion, ribosome binding site, polyadenylation sequence, a multi-linker region for inserting nucleic acids encoding antibodies to be expressed and an optional marker element.

Host cells can be prokaryotic cells, such as bacterial cells; or lower eukaryotic cells, such as yeast cells; or higher eukaryotic cells, such as mammalian cells. Representative examples are: Escherichia coli, Streptomyces; bacterial cells of Salmonella typhimurium; fungal cells such as yeast; insect cells of Drosophila S2 or Sf9; animal cells of CHO, COS7, 293 cells, etc.

In some embodiments, host cells may be various functional cells well known in the art, such as various killer cells, including but not limited to cytokine induced killer cells (CIK), dendritic cell stimulated cytokine induced killer cells (DC-CIK), cytotoxic T lymphocytes (CTL) γδ T cells, natural killer cells (NK), tumor infiltrating lymphocytes (TIL), lymphokine activated killer cells (LAK), CD3AK cells (killer cells of anti-CD3 mAb), and car-t/tcr-t cells. In some embodiments, the killer cells are T cells or NK cells. Exemplary NK cells include, but are not limited to, primary NK cells, NK cell strains (such as NK92), and NKT cells. In some embodiments, the NK cells are primary NK cells. Exemplary T cells include, but are not limited to, peripheral blood T lymphocytes, cytotoxic T cells (CTLs), helper T cells, inhibitory / regulatory T cells γδ T cells and T cells with mixed cell populations such as cytokine induced killer cells (CIK) and tumor infiltrating lymphocytes (TIL). In some embodiments, the T cells are selected from peripheral blood T lymphocytes and til derived T cells.

Transformation of host cells with recombinant DNA can be performed using conventional techniques familiar to those skilled in the art. When the host is a prokaryote such as Escherichia coli, competent cells that can absorb DNA can be harvested after the exponential growth period and treated with CaCl₂ method. The steps used are well known in the art. Another method is to use MgCl₂. If necessary, the transformation can also be carried out by electroporation. When the host is eukaryote, the following DNA transfection methods can be selected: calcium phosphate coprecipitation method, conventional mechanical methods such as microinjection, electroporation, liposome packaging, etc.

The obtained transformants can be cultured by conventional methods to express the polypeptide encoded by the gene of the description. According to the host cells used, the medium used in the culture can be selected from various conventional media. Culture is performed under conditions suitable for host cell growth. When the host cells grow to the appropriate cell density, the selected promoters are induced by appropriate methods (such as temperature conversion or chemical induction), and the cells are cultured for another period of time.

The polypeptide in the above method can be expressed inside the cell, on the cell membrane, or secreted outside the cell. If necessary, the recombinant protein can be separated and purified by various separation methods using its physical, chemical, and other characteristics. These methods are familiar to those skilled in the art. Examples of these methods include but are not limited to: conventional renaturation treatment, treatment with protein precipitant (salting-out method), centrifugation, permeation, ultra-treatment, ultra-centrifugation, molecular sieve chromatography (gel filtration), adsorption chromatography, ion exchange chromatography, high-performance liquid chromatography (HPLC) and other various liquid chromatography technologies and combinations of these methods.

### Therapeutic use and pharmaceutical composition

By constructing a nanoantibody library, the inventor discovered, expressed and purified a number of nanoantibodies that can bind EGFR protein. The binding ability and drug safety of these antibodies were verified by protein level affinity detection, protein binding epitope analysis, ligand competition test, species cross test, cell level affinity detection, tumor cell EGFR binding detection, membrane proteome array screening and tissue cross reaction.

Therefore, all aspects of the antibodies described herein can be used to prepare drugs to prevent or treat various conditions and diseases described herein, especially those related to cells expressing human epidermal growth factor receptor. In some embodiments, the condition and disease are cancers, including but not limited to head and neck cancer, breast cancer, bladder cancer, ovarian cancer, renal carcinoma, colon cancer, non-small cell lung cancer, and the like.

The pharmaceutical composition herein comprises the binding molecules described herein, as well as pharmaceutically acceptable excipients, including but not limited to diluents, vehicles, solubilizers, emulsifiers, preservatives, and/or adjuvants. The excipients are preferably non-toxic to the recipient at the dose and concentration used. Such excipients include (but are not limited to): saline, buffer, glucose, water, glycerol, ethanol, and their combinations. In some embodiments, the pharmaceutical composition may contain substances for improving, maintaining, or retaining, for example, the pH, permeability, viscosity, clarity, color, isotonicity, odor, sterility, stability, dissolution or release rate, absorption, or permeation of the composition. These substances are known in the prior art. The optimal pharmaceutical composition can be determined according to the expected route of administration, mode of delivery, and required dose.

Pharmaceutical compositions for in vivo administration are usually provided in the form of sterile formulations. Sterilization is achieved by filtration through a sterile filter membrane. When the composition is lyophilized, this method can be used for sterilization before or after lyophilization and rehydration. The pharmaceutical composition of the present description may be selected for parenteral delivery. Compositions for parenteral administration may be in lyophilized form or stored in solution. For example, it is prepared by conventional methods with normal saline or aqueous solution comprising glucose and other adjuvants. Parenteral compositions are usually placed in containers with sterile access holes, such as intravenous solution strips or vials with plugs that can be pierced by subcutaneous injection needles. Alternatively, compositions may be selected for inhalation or delivery through the digestive tract, such as oral. The preparation of the pharmaceutically acceptable composition is within the art. Other pharmaceutical compositions will be apparent to those skilled in the art, including formulations comprising antibodies in sustained or controlled release delivery formulations. The techniques used to prepare a variety of other sustained or controllable delivery modes (such as liposome carriers, bioerodible particles or porous beads, and deposit injection) are also known to those skilled in the art.

Once the pharmaceutical composition is prepared, it is stored in sterile vials in the form of solution, suspension, gel, emulsion, solid, crystal or dehydrated or lyophilized powder. The formulation may be stored in ready to use form or rehydrated before administration (e.g., lyophilized). The description also provides a kit for generating a single dose administration unit. The kit of the description can each contain a first container with dried protein and a second container with aqueous formulation. In some embodiments of the present description, a kit comprising single-chamber and multi-chamber pre-filled syringes (e.g., liquid syringes and lyophilized syringes) are provided.

The present description also provides a method for treating a patient (especially a patient's EGFR related disease) by administering a binding molecule or a pharmaceutical composition thereof according to any embodiment of the present description. Terms "patient", "subject", "individual" and "object" are used interchangeably herein, including any organism, preferably animals, more preferably mammals (such as rats, mice, dogs, cats, rabbits, etc.), and most preferably humans. "Treatment" refers to the subject accepting the treatment scheme described herein to achieve at least one positive treatment effect (for example, a reduced number of cancer cells, a reduced tumor volume, a reduced rate of cancer cells infiltrating into peripheral organs, or a redeuced rate of tumor metastasis or tumor growth). The treatment scheme for effectively treating patients can vary according to many factors, such as the patient's disease status, age, weight, and the ability of the therapy to stimulate the subject's anti-cancer response.

The therapeutically effective amount of the pharmaceutical composition comprising the binding molecule of the present description to be used will depend on, for example, the degree and the target of treatment. Those skilled in the art will understand that the appropriate dose level for treatment will vary in part depending on the molecule delivered, the indication, the route of administration, and the size (body weight, body surface or organ size) and/or condition (age and general health condition) of the patient. In some embodiments, clinicians may titrate the dose and change the route of administration to obtain the optimal therapeutic effect. For example, about 10 micrograms/kg body weight per day to about 50 mg/kg body weight.

The dosing frequency will depend on the pharmacokinetic parameters of the bound molecules in the formulation used. Clinicians typically administer the compositions until a dosage that achieves the desired effect. The composition may therefore be administered as a single dose, or over time as two or more doses (which may or may not contain the same amount of the desired molecule), or as a continuous infusion through an implanted device or catheter.

The route of administration of the pharmaceutical composition is according to known methods, such as oral, intravenous, intraperitoneal, intracerebral (intraparenchymal), intraventricular, intramuscular, intraocular, intra-arterial, portal vein or intralesional injection; by continuous release system or by implantable device.

### Diagnosis, Detection, and Kit

The binding molecule of the present description can be used in assays due to its high avidity with EGFR, such as binding assays to detect and/or quantify EGFR expressed in tissues or cells. Binding molecules such as single domain antibodies can be used in further studies investigating the function of EGFR in disease. The methods for detecting EGFR are roughly as follows: obtaining cell and/or tissue samples; and detecting the level of EGFR in the sample.

The human epidermal growth factor receptor binding molecule of the invention can be used for diagnostic purposes to detect, diagnose or monitor diseases and / or conditions related to human epidermal growth factor receptor. The invention provides a method for detecting the presence of human epidermal growth factor receptor in a sample using a classical immunohistological method known to those skilled in the art. Human epidermal growth factor receptor can be detected in vivo or in vitro. Examples of methods suitable for detecting the presence of human epidermal growth factor receptor include ELISA, FACS, RIA, etc.

For diagnostic uses, binding molecules such as single domain antibodies are usually labeled with detectable labeling groups. Suitable labeling groups include (but are not limited to): radioisotopes or radionuclides (e.g., 3H, 14C, 15N, 35S, 90Y, 99Tc, 111In, 125I, 131I), fluorescent groups (e.g., FITC, rhodamine, lanthanide phosphors), enzymatic groups (e.g., horseradish peroxidase, β-galactosidase, luciferase, alkaline phosphatase), chemiluminescent groups, biotinyl groups, or predetermined polypeptide epitopes recognized by secondary reporters (e.g., leucine zipper pair sequences, binding sites for secondary antibodies, metal binding domains, epitope tags), MRI (magnetic resonance imaging) or CT (computed tomography) contrast agents. Various methods for labeling proteins are known in the art and can be used to carry out the present description.

Another aspect of the present description provides a method for detecting the presence of a test molecule that competes with an antibody of the present description to bind EGFR. An example of one such assay would involve detecting the amount of free antibody in a solution comprising an amount of EGFR in the presence or absence of the test molecule. An increase in the amount of free antibody (i.e., antibody that does not bind EGFR) will indicate that the test molecule can compete with the antibody for binding to EGFR. In one embodiment, the antibody is labeled with a labeling group. Alternatively, the test molecule is labeled, and the amount of free test molecule is monitored in the presence or absence of the antibody.

The description also provides a detection kit for detecting EGFR level. The kit comprises an antibody that recognizes EGFR protein, a lysis medium for dissolving samples, and general reagents and buffers required for detection, such as various buffers, detection markers, detection substrates, etc. The detection kit can be an in vitro diagnostic device.

The present invention will be described below in the form of specific examples. It should be understood that these examples are merely illustrative and are not intended to limit the scope of the present description. Unless otherwise specified, the methods and materials used in the examples are conventional materials and methods in the art.

### Example

### Example 1. Immunization of Alpaca

### 1.1 Preparation of Immunogen:

The sequence of EGFR protein (L25-S645, P00533-1 )was obtained from uniprot, and was fused with the sequence of human IgG Fc fragment, and the eukaryotic expression vector of pCDNA3.4 (Thermo) plasmid was synthesized and constructed by Nanjing Genscript Company. The synthesized plasmid was expressed using ExpiCHO^{™} (Thermo Fisher) expression system. After expression, 5 ml of protein A pre-packed column (GE) was used for one-step affinity purification, and the purified sample was replaced into PBS buffer. After the purity was identified by SDS-PAGE electrophoresis gel and HPLC, and the activity was identified by ELISA, the sample was split and frozen in -80°C refrigerator for subsequent immunization.

### 1.2 Immunization of Alpaca:

For first immunization, 400 µg of antigen was mixed with the adjuvant (GERBU FAMA). An alpaca was immunized subcutaneously at four sites on the back with an immune amount of 1 mL at each site. The second to sixth the immunization: the amount of immune antigen was 200 µg, and the alpaca was immunized subcutaneously at four sites on the back with an immune amount of 1 mL at each site, the interval of each immunization being one week.

### 1.3 Detection of Immune Serum Titer:

### 1.3.1 Detection of Titer at Protein Level

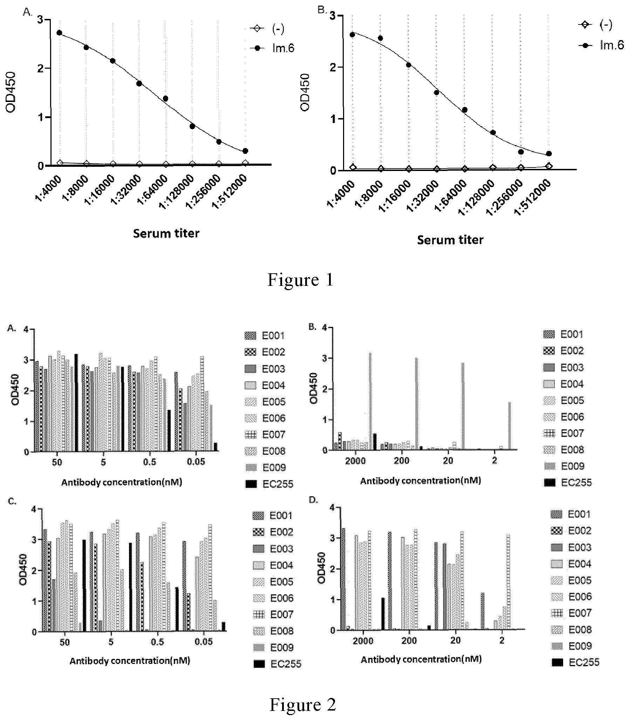

EGFR.His antigen (Aero, Cat#EGR-H522a) , EGFR-V3-his tag antigen (Aero, Cat#EGI-H52H4 )were coated overnight at 4 degrees. After blocking and washing, the gradient diluted serum was added to the ELISA plate for incubation, and then incubated with the anti-llama IgG HRP (Abcam) antibody. After washing, TMB chromogenic solution was added for development, and the reaction was terminated with 2 M HCl. Then the absorbance value at OD450 nm was detected with a microplate reader. As shown in Figure 1, the titer of Alpaca reached a high level (>512000) after six immunizations. A and B in Figure 1 show the binding titers of Alpaca serum before and after immunization with EGFR.His and EGFR-V3-his tag antigens respectively.

### Example 2. Construction and Screening of Nanobody Immune Library for EGFR

(1) After six immunizations, 100 mL of camelid peripheral blood lymphocytes were extracted, and total RNA was extracted. RNA was extracted according to the instructions of RNAiso reagent of Takara.
(2) The first strand of cDNA was synthesized with RNA as template and oligo dT as primer according to the instructions of reverse transcriptase of Takara.
(3) The variable region coding gene of heavy chain antibody was obtained by nested PCR using PrimeSTAR high fidelity DNA polymerase. The variable region fragment of the heavy chain antibody was amplified by nested PCR:
   First round of PCR:
      Upstream primer: GTCCTGGCTGCTCTTCTACAAGGC (SEQ ID NO: 84 )
      Downstream primer: GGTACGTGCTGTTGAACTGTTCC (SEQ ID NO: 85 )
      The fragment between the heavy chain antibody guide peptide and antibody CH2 was amplified, annealed at 55°C for 30 cycles; a DNA fragment of about 600 bp was recovered as a template for the second round of PCR.
   Second round of PCR:
      Upstream primer:GATGTGCAGCTGCAGGAGTCTGGRGGAGG (SEQ ID NO: 86 )
      Downstream primer: GGACTAGTGCGGCCGCTGGAGACGGTGACCTGGGT (SEQ ID NO: 87 )
      The fragment (long fragment and short fragment) between the FR1 region and the long and short hinge regions of the heavy chain antibody was amplified, annealed at 55°C for 30 cycles, and the target fragment was recovered. The result showed that the size of the fragment was about 500 bp, that is, the nanobody gene electrophoresis band was about 500 bp.
(4) The phage pME207 and PCR amplification products were digested with Sfi I and Not I (NEB), respectively. After recovery and quantification, the two fragments were ligated with T4 DNA ligase (Takara) at a molar ratio of 1:3 and ligated overnight at 16°C.
(5) After ethanol precipitation, the ligated product was dissolved in 100 µL sterile water and electroporated into Escherichia coli TG1 in ten times. 100µL of the bacterial solution was taken after electric shock and culture, diluted by multiple ratio, coated on an ampicillin LB culture plate, the storage capacity was calculated, and the rest was coated with ampicillin 2×YT culture plate, at 37°C, invertly cultured for 13-16 h. After scraping and washing the colonies on the culture plate with 10 ml, 2×YT medium, 25% glycerol at the final concentration was added, split, and stored at -80°C for further use. The size of the storage capacity is 4.3 × 10⁹. To detect the insertion rate of the library, 48 clones were randomly selected for colony PCR, and the results showed that the insertion rate had reached more than 90%.
(6) According to the calculated library capacity results, viable cells with 10 times the library capacity were seeded in 200ml of 2×YT (comprising 2% glucose, 100 µg/ml ampicillin), cultured at 37°C for 200 r/min until the OD600 reached 0.5, auxiliary phage was added according to the multiplicity of infection of 20:1, and left for 30 min at 37°C, 200 r/min for 30 min. The culture was centrifuged, and the pellet was resuspended with 200 ml of 2×YT (comprising 100 µg/ml ampicillin and 50 µg/ml kanamycin), incubated overnight at 37°C, 250 r/min, centrifuged at 8000rpm to obtain the supernatant, added with 5×PEG/NaCl solution, placed on ice for 60 min, centrifuged at 8000rpm for 30 min. The pellet was resuspended in 5 ml of PBS to obtain the single domain heavy chain antibody (VHH) immune library against EGFR, and 10 µL was taken to determine the titer, and the rest were split at -80°C for storage.
(7) Using EGFR.His protein was selected by phage library panning; The protein was coated on the microplate plate at 2µg/mL and 100 µL per well, and placed overnight at 4 °C. The next day, 200 µL, 3% BSA were added to the five wells and blocked for 2 hours at room temperature. Two hours later, they were washed three times with PBST (containing 0.05% Tween 20 in PBS). After washing the plate, first add 100 µL of phage pre blocked with 5% skim milk (2~3×10¹¹ tfu immune camel nano antibody phage display Gene Library) to each negative screen hole, and act for 1.5 hours at room temperature, and then transfer the supernatant after negative screen to the target antigen coating hole, and act for 1.5 hours at room temperature. Wash with PBST (containing 0.05% Tween 20 in PBS) for 12 times to wash out the unbound phages. The phage specifically bound to EGFR was dissociated with glycine (sigma), and the eluted phage was neutralized by tris (Invitrogen, 1M, pH 8.0) and infected with TG1 in logarithmic phase. After propagation and expansion, the next round of "adsorption elution" was carried out. Finally, the eluted phage was impregnated with TG1, and the expression of nano antibody was induced by IPTG (thermo). The ELISA plate was coated with EGFR protein and EGFR-DIII- AlpFc protein. The supernatant was taken for ELISA detection, and the clones with OD450>0.5 were selected for sequencing.
(8) After sequence analysis, a total of 182 clones that could bind EGFR protein were obtained.

### Example 3. Expression and Purification of Candidate Antibodies

9 nano antibody VHH sequences with high binding activity to EGFR (see tables 6 and 7 for their numbers and CDR1, CDR2, CDR3 and VHH sequences) were selected and constructed on pCDNA3.4-IgG4 vector to form VHH-IgG4, and then expressed by ExpiCHO^{™} (Thermo Fisher) expression system. After one week of expression, the supernatant was collected for protein A (GE) purification. Then Nanodrop was used to detect the protein quality, and HPLC was used to detect the protein purity. The purity and yield of the obtained protein met the requirment of subsequent tests.

### Example 4. Characterization of Candidate Antibodies

(1) Detection of Affinity at Protein Level
The binding kinetics and affinity of antibody to human EGFR.His antigen were determined using surface plasmon resonance (SPR). The purified antibody was flowed through the sensor chips pre-immobilized with protein A, and the antibody was captured by protein A. Then five different concentrations of EGFR.His protein were used as the mobile phase, and the binding time and dissociation time were 30min and 60min, respectively. The binding rate (ka), dissociation rate (kd), and equilibrium constant (kd) were analyzed using Biacore Evaluation Software 2.0 (GE). EC225, an antibody that binds to the III region of human EGFR, was selected as a positive control (sequence source https://go.drugbank.com/drugs/DB00002). The SPR detection results are shown in Table 1 .

**Table 1. Affinity Detection Results of Antibodies**

| Antibodies | ka (1/Ms) | kd (1/s) | KD (M) |
|---|---|---|---|
| E001 | 5.79E+04 | 1.20E-05 | 2.06E-10 |
| E002 | 4.85E+04 | 1.21E-04 | 2.49E-09 |
| E003 | 2.84E+04 | 1.33E-04 | 4.68E-09 |
| E004 | 1.50E+05 | 7.76E-04 | 5.17E-09 |
| E005 | 1.64E+04 | 4.67E-05 | 2.86E-09 |
| E006 | 3.74E+04 | 2.55E-04 | 6.82E-09 |
| E007 | 3.71E+04 | 1.08E-05 | 2.92E-10 |
| E008 | 1.53E+06 | 1.48E-02 | 9.62E-09 |
| EC255 | 1.54E+06 | 4.58E-04 | 2.97E-10 |

(2) Protein binding epitope analysis: ELISA was used to determine the binding of VHH-IgG4 antibody to EGFR extracellular proteins. Briefly, EGFR his tag antigen , EGFR-V3-his tag antigen protein were diluted with PBS to 1 µg/mL, EGFR-D1D2 (L25-A310, P00533-1 (uniprot) ), EGFR-D4 (F481-S645, P00533-1 (uniprot)) his tag antigen were diluted with PBS to 0.5 µg/mL, and each well of an ELISA plate was filled with 100 µL antigen and coated overnight at 4 °C. The next day, the plates were washed twice with PBST (added with 0.5% TWEEN-20), then blocked with 3 %BSA at 37 °C for 1.5 h, and then washed twice with PBST. Then, the antibody to be tested was diluted to 50nM, 5 nM, 0.5 nM, 0.05 nM according to 100µL/ well into the plate wells coated with EGFR his tag protein and EGFR-V3-his tag antigen respectively; The antibody was diluted to 2000nM, 200 nM, 20 nM, 2nM according to 100µL/ well into the plate wells coated with EGFR his tag protein and EGFR-V3-his tag antigen respectively; The ELISA plates were incubated at 37°C for 1H. The plates were then washed three times with PBST, and then 100µL HRP labeled Goat anti human IgG Fc antibody (1:10000, Bethyl) and incubated at 37°Cfor another 40min. The plates were then washed four times with PBST according to 100µL/ well, add TMB chromogenic solution, and react for 3 ~ 5min; According to 100µL/ well, 3N hydrochloric acid was added to stop the reaction. After the reaction was terminated, OD450/650 was selected on the Tecan microplate reader for detection. The results are shown in Fig. 2 and table 2. A-D in Fig. 2 respectively show the binding of each antibody to EGFR his tag protein, EGFR-D1D2 protein, EGFR-V3-his tag, and EGFR-D4 protein. Except for E009 antibody binding to region I+II, other antibodies bind to regions III and IV of EGFR extracellular segment.

**Table 2.**

| Antibody number | Binding region |
|---|---|
| E001 | IV |
| E002 | III |
| E003 | III&IV |
| E004 | IV |
| E005 | IV |
| E006 | IV |
| E007 | IV |
| E008 | III |
| EC255 | III |
| E009 | I+II |

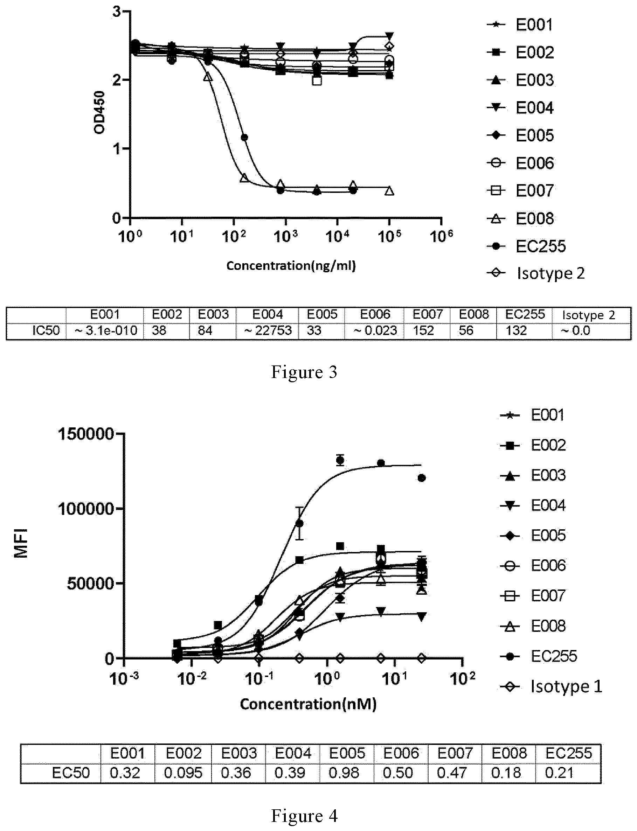

(3) Ligand Competition Assay
ELISA was used to identify whether the VHH-IgG4 antibody competed with the ligand EGF for binding to EGFR extracellular protein. Briefly, EGFR his tag protein was diluted with PBS to 2µg/mL, and 100µL antigen was coated overnight at 4 °C. The next day, the plates were washed twice with PBST (added with 0 .5 % TWEEN-20), then blocked with 3%bsa at 37°C for 1.5h, and then washed twice with PBST. Human EGF protein, mouse IgG2a FC tag (Aero, Cat#EGF-H525b, 100µg) protein was diluted to 10µg/ mL. The VHH-IgG4 antibody was diluted to 2000nM, and then 4-fold diluted for 8 gradients. Add 50 µL EGF protein and 50 µL diluted antibody to each well, and the enzyme plate was incubated at 37 °C for 1H. After washing for three times, HRP Goat anti mouse IgG (H+L) antibody (Abeam, cat#ab205719) diluted at 1:3000 was added to each well and incubated at 37 °C for 40min. Then the plates were washed three times with PBST according to 100µL/ well, add TMB chromogenic solution, and react for 3 ~ 5min. According to 100µL/ well, 3N hydrochloric acid was added to stop the reaction. After the reaction was terminated, OD450/650 was selected on the Tecan microplate reader for detection. The dose-response curve was made with Graphprism 8.0 software, and the IC50 was calculated. Isotype control 2 is the negative control, and its amino acid sequence is shown in SEQ ID NO:2 in CN106046152A. The results are shown in Figure 3.
(4) Cross-Species Reactivity Assay
ELISA was used to identify the binding of VHH-IgG4 antibody to mouse EGFR, monkey EGFR and human EGFR protein extracellular segments. The detection steps were as follows: dilute mouse EGFR protein, his tag protein ( (Acro, Cat#EGR-C52H1), rhesus macaque EGFR protein, His Tag (Aero, Cat#EGR-C52H1 ) to 1µg/mL, followed by 100µL per well was added to 96 well enzyme labeled plate and coated overnight at 4 °C; The next day, the plates were washed twice with PBST (added with 0.5 % TWEEN-20), then blocked with 3% BSA at 37°C for 1.5h, and then washed twice with PBST. The VHH-IgG4 antibody was diluted to 50nM, add 100µL to each well, incubate at 37 °C for 1H; The plates were then washed three times with PBST, and then 100µL HRP labeled Goat anti human IgG Fc antibody (1:10000,Bethyl) and incubated at 37°Cfor another 40min. The plates were then washed four times with PBST according to 100µL/ well, add TMB chromogenic solution, and react for 3 ~ 5min; According to 100µL/ well, 3N hydrochloric acid was added to stop the reaction. After the reaction was terminated, OD450/650 was selected on the Tecan microplate reader for detection. The results are shown in Table 3 below. All the proteins in binding region III bind to mouse EGFR protein.

**Table 3: summary of Cross-Species Reactivity Assay (OD450 )**

| Antibody number | Mouse EGFR | Monkey EGFR |
|---|---|---|
| E001 | 0.76 | 3.30 |
| E002 | 2.73 | 3.06 |
| E003 | 2.80 | 3.15 |
| E004 | 3.37 | 3.64 |
| E005 | 3.33 | 3.19 |
| E006 | 3.13 | 3.30 |
| E007 | 3.21 | 3.35 |
| E008 | 0.72 | 3.68 |
| E009 | 0.08 | 3.36 |
| EC255 | 0.11 | 3.56 |

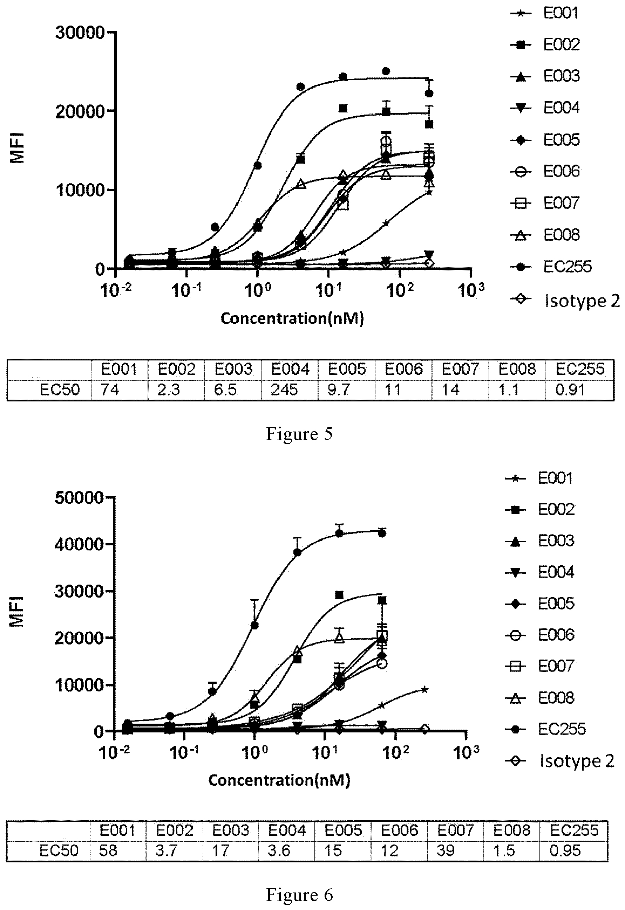

(5) Cell level affinity assay:
Will express EGFR III Region HEK293T cells (i.e., HEK293T EGFR- III Cells) were plated in 96 well plates with 3×10⁵ cells per well, and then HEK293T EGFR- III cells was incubated with gradient diluted VHH-IgG4 antibody. After incubation for half an hour, anti human IgG PE (Jackson Immuno Research, Code: 109-117-008, Lot:145501) was added for incubation, and then CytoFLEX flow cytometry was used for detection. The EC50 of antibody was calculated by curve fitting with Graphprism8.0 software. The results are shown in Figure 4. The isotype control 1 in the figure is a negative control, and its amino acid sequence is shown in SEQ ID NO. 2 in CN 106146653A.
(6) Detection of EGFR binding to tumor cells:
SKOV3 and Aspc-1 tumor cells expressing EGFR were respectively plated in 96 well plates, with 3×10⁵ cells per well, and then the tumor cells were incubated with gradient diluted VHH-IgG4 antibody. After incubation on ice for half an hour, anti human IgG PE (Jackson Immuno Research, Code: 109-117-008, Lot:145501) was added for incubation, and then CytoFLEX flow cytometry was used for detection. The EC50 of the antibody was calculated by fitting the curve. The results are shown in Figure 5 and Figure 6, respectively.

### Example 5: Membrane Proteome Array Screening

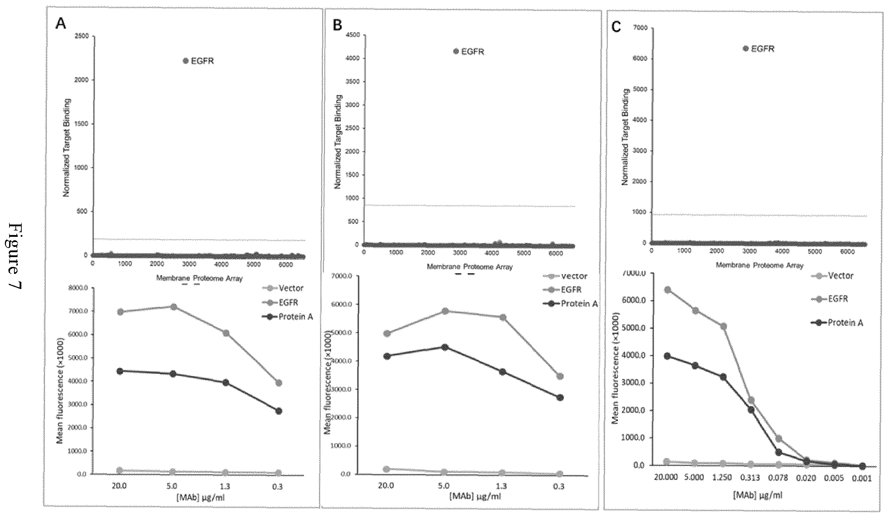

Membrane Proteome Array (MPA) is a membrane protein array screening platform developed by American Integral molecular company. They display 5300 different human membrane proteins on the cell surface by transfecting HEK293 cells. The binding signal of antibody on these proteins is detected by FACS, so as to evaluate the specificity of the antibody to be detected. The MPA screening results showed that E002, E005 and E008 had good specificity (as shown in A, B and C of Figure 7 respectively), and only specifically bound to the target protein EGFR.

### Example 6: Tissue Cross-Reactivity

This trial was mainly completed by the clinical trial testing center under Shanghai cell therapy group. The purpose of this study is to evaluate whether several preferred antibodies cross react with 12 or 34 kinds of normal human frozen tissues (each tissue is derived from 3 different individuals) by using the immunohistochemical staining method of streptavidin combined with biotin. The process is as follows: select 12 or 34 kinds of tissues to make frozen sections, dry them at room temperature and fix them with acetone. Blocking was performed using reagent A and reagent B of the endogenous biotin blocking Kit (Sangon, E674001). Biotin labeled antibody samples were incubated for 30min, and horseradish peroxidase labeled streptavidin (Abeam, ab7403) was added to incubate for 15min after washing. DAB color development, hematoxylin counterstaining, neutral plastic sealing, natural air drying and microscopic examination. The positive control was anti EGFR antibody (Biotin)(Abcam), and the negative control was biotin labeled IgG isotype control.

Table 4 shows the results of tissue cross reaction between 9 EGFR targeting VHH-IgG4 antibodies and 12 normal human tissues. The 9 EGFR nanoantibodies and the purchased commercial EGFR antibodies have obvious localization and typical positive reaction on tonsil, squamous epithelium of cervix, skin and placenta. The cross reaction range of E008 antibody was relatively minimal and weak; In addition to cross reaction with tonsil, squamous epithelium of cervix, skin and placenta, other antibodies also cross react with liver, kidney, gallbladder and other tissues to varying degrees. Among them, E007 antibody has the widest and strongest tissue cross reaction.

Table 5 shows the tissue cross reaction results of three EGFR targeting VHH-IgG4 antibodies with 32 normal human tissues. The results of cross reaction of three antibody tissues were almost the same, without much difference. The positive reaction was localized in endothelial cells or squamous epithelial cells with positive control antibody reaction. E008 showed less tissue cross reactivity than the other two antibodies.

**Table 4: Tissue cross reactivity of 9 EGFR antibodies with 12 normal human tissues**

| | tonsi l | cerv ix | place nta | thyroid | breast | lung | liver | kidne y | duo den um | uter ine bod y | sto mac h | gallb ladde r |
|---|---|---|---|---|---|---|---|---|---|---|---|---|
| **Positive control** | positiv e (5%) | Squa mous epithe lium positi ve (5%) | Strong positive (>75%) | - | - | - | - | - | - | - | - | Atypic al positiv ity is rarely seen |
| **negativ e control** | **-** | - | - | - | - | - | - | - | - | - | - | - |
| **E001** | positiv e (5%) | Squa mous epithe lium positi ve (5%) | Strong positive (>75%) | - | - | - | Atypica l positive (25%) | Weak positive (>=5% ) | - | - | - | |
| **E002** | positiv e (5%) | Squa mous epithe lium positi ve (5%) | Strong positive (>75% ) | Occasion al atypical positive | Occasion al atypical positive (2%-3%) | Occas ional atypic al positi ve | Occasio nal atypical positive (2%) | Weak positive (3%) | - | - | Atyp ical posit ivity (stro ma) is rarel y seen | Atypic al positiv ity is rarely seen |
| **E003** | positiv e (5%) | Squa mous epithe lium positi ve (5%) | Strong positive (>75% ) | - | Atypical positivity is rarely seen | Occas ional atypic al positi ve | Extrem ely occasio nal positive | Occasio nally positive | - | - | - | Atypic al positiv ity is rarely seen |
| **E004** | positiv e (5%) | Squa mous epithe lium positi ve (5%) | Strong positive (>75% ) | - | - | - | Atypica l weak positive (3%) | Weak positive (3%) | - | - | - | Atypic al positiv ity is rarely seen |
| **E005** | positiv e (5%) | Squa mous epithe lium positi ve (5%) | Strong positive (>75% ) | Atypical weak positive (3%-5%) | Occasion al atypical positive | Occas ional atypic al positi ve | Atypica l moderat e positive (>25% ) | Weak positive (10%) | - | - | - | Atypic al positiv ity is rarely seen |
| **E006** | positiv e (5%) | Squa mous epithe lium positi ve (5% ) | Strong positive (>75% ) | - | - | - | - | Weak positive (2%) | - | - | - | Atypic al positiv ity is rarely seen |
| **E007** | positiv e (5%) | Squa mous epithe lium positi ve (5%) | Strong positive (>75% ) | Atypical weak positive (5%) | Atypical weak positive (5%) | Atypi cal weak positi ve (5%) | Atypica l strong positive (>75% ) | Strong positive (>75% ) | - | - | - | Atypic al positiv ity is rarely seen |
| **E008** | positiv e (5%) | Squa mous epithe lium positi ve (5%) | Strong positive (>75% ) | - | - | - | - | Extrem ely occasio nal positive | - | - | - | - |
| **E009** | positiv e (5%) | Squa mous epithe lium positi ve (5% ) | Strong positive (>75% ) | - | Atypical positivity is rarely seen | - | Occasio nally positive | Weak positive (3%) | - | - | - | Atypic al positiv ity is rarely seen |
| **EC255** | positiv e (5%) | Squa mous epithe lium positi ve (5%) | Strong positive (>75% ) | Occasion al atypical positive | Occasion al atypical positive | - | Atypica l moderat e positive (>25% ) | Weak positive (10%) | - | - | - | Atypic al positiv ity is rarely seen |

**Table 5: Tissue cross reactivity of three EGFR antibodies with 32 normal human tissues**

| Numbe r | Organ | Positive Control | Negativ e Control | **E004** | **E006** | **E008** |
|---|---|---|---|---|---|---|
| 1 | tonsil | positive (5%) | - | positive (5%) | positive (5%) | positive (5%) |
| 2 | cervix | Squamous epithelium positive (5%) | - | Squamous epithelium positive (5%) | Squamous epithelium positive (5%) | Squamous epithelium positive (5%) |
| 3 | placenta | Strong positive (>75%) | - | Strong positive (>75%) | Strong positive (>75%) | Strong positive (>75%) |
| 4 | thyroid | - | - | - | - | - |
| 5 | breast | - | - | - | - | - |
| 6 | lung | - | - | - | - | - |
| 7 | liver | - | - | Atypical weak positive (3%) | - | - |
| 8 | kidney | - | - | Weak positive (3%) | Weak positive (2%) | Extremely occasional positive |
| 9 | duodenum | - | - | - | - | - |
| 10 | uterine body | - | - | - | - | - |
| 11 | stomach | - | - | - | - | - |
| 12 | gallbladde r | Atypical positivity is rarely seen | - | Atypical positivity is rarely seen | Atypical positivity is rarely seen | - |
| 13 | colon | - | - | - | - | - |
| 14 | prostate | positive | - | positive | positive | positive |
| 15 | artery | - | - | - | Occasionally positive | - |
| 16 | parathyroi d gland | positive | - | positive | positive | positive |
| 17 | ovary | - | - | - | - | - |
| 18 | cerebellu m | Occasionally positive | - | Weak positive | Weak positive | Weak positive |
| 19 | fallopian tube | Occasionally positive | - | Occasionally positive | Occasionally positive | Extremely occasional positive |
| 20 | heart | Endothelium positive | - | - | - | - |
| 21 | ureter | - | - | Occasionally positive | Occasionally positive | Occasionally positive |
| 22 | skeletal muscle | - | - | Occasionally positive | Occasionally positive | Occasionally positive |
| 23 | testis | Endothelium positive | - | Endothelium positive | Endothelium positive | Endothelium positive |
| 24 | skin | Epithelial positivity | - | Epithelial positivity | Epithelial positivity | Epithelial positivity |
| 25 | Ileum | - | - | - | - | - |
| 26 | adrenal gland | Occasionally positive | - | Occasionally positive | Occasionally positive | Occasionally positive |
| 27 | bladder | Occasionally positive | - | Occasionally positive | Occasionally positive | Occasionally positive |
| 28 | spinal cord | - | - | - | - | - |
| 29 | left eye | Endothelium positive | - | Endothelium positive | Endothelium positive | Endothelium positive |
| 30 | pituitary | - | - | Occasionally positive | Occasionally positive | Occasionally positive |
| 31 | spleen | - | - | Occasionally positive | Occasionally positive | - |
| 32 | brain | - | - | - | - | - |

### Example 7: Summary of VHH Antibody Sequences

The CDR sequences of 9 VHH antibodies are shown in Table 6, and the amino acid sequences of 9 VHH antibodies and EC255 control antibody are shown in Table 7.

**Table 6: Summary of VHH antibody CDR sequences**

| Antibody number | CDR1 | SEQ ID NO: | CDR2 | SEQ ID NO: | CDR3 | SEQ ID NO: |
|---|---|---|---|---|---|---|
| E001 | GSGFTIQA | 4 | IHQGGST | 13 | NWPPLRV | 22 |
| E002 | GFTFSDYA | 5 | ITGTGETS | 14 | SIYHPPRDY | 23 |
| E003 | GGVFSSYA | 6 | ITSGHST | 15 | NRSPYTPGN | 24 |
| E004 | GFPFETYA | 7 | IFPDGKHT | 16 | AKDLPFFATPSFYIGY | 25 |
| E005 | GFAFSDYT | 8 | ITSAGDII | 17 | NALVSASVRASDY | 26 |
| E006 | GFTFDTYT | 9 | LATTGGK P | 18 | KATSRSPVNPHNY | 27 |
| E007 | GFSFSDHT | 10 | ITPAGDII | 19 | NALVSAAVRASDY | 28 |
| E008 | GLPLLDYA | 11 | ISISEGST | 20 | AADKTDIGFWLLVGG AGGGEVRYEY | 29 |
| E009 | GRIDIIFT | 12 | VTNYNA | 21 | HLNYASDYTEYDY | 30 |

**Table 7: Summary of amino acid sequence of VHH antibody**

| Antibod y number | VHH amino acid sequence | SEQ ID NO: |
|---|---|---|
| E001 | EVQLVESGGGLVQPGGSLRLSCTPSGSGFTIQAMTWYRQAPGKQRELVAIIHQGGST DYSDSVRGRFTISRDNAKTAWYLQMNNLKPEDTAVYYCNVVPPLRVWGQGTQVT VSS | 31 |
| E002 | QVQLVESGGGLVQAGGSLRLSCAASGFTFSDYAVSWYRQAPGKEREFVARITGTGE TSRYEDSVKGRFAISRDNVKNTAYLQMNSLKPEDTAVYYCSIYHPPRDYWGQGTQV TVSS | 32 |
| E003 | QVQLVESGGGLVQPGGSLRLSCVASGGVFSSYAMGWYRQVPGKLRELVATITSGHS TTYADIVRGRFTISRDNTKNTVYLQMNRLEPDDTAVYYCNRSPYTPGNWGQGTLVT VSS | 33 |
| E004 | EVQLVESGGGLAQSGGSLRLACEASGFPFETYAMSWARQTPGNEPEWVAGIFPDGK HTAYAHSVKGRFTISRVNANNTVYLQMDSLKPEDTAVYYCAKDLPFFATPSFYIGY WGQGTLVTVSS | 34 |
| E005 | QVQLVESGGGLVQPGGSLRLSCEASGFAFSDYTMRWHRQAAGKELELVAYITSAGD IIKYANSVRGRFTISRDNAKNTLYLQMDSLKPEDTAVYRCNALVSASVRASDYWGQ GTLVTVSS | 35 |
| E006 | EVQLVESGGGLVQPGGSLRLSCTASGFTFDTYTTSWERQAPGKEREMVAVLATTGG KPYYVESVKGRFTISRDASETAVTLQMNNLKPEDTAVYYCKATSRSPVNPHNYSGQ GTLVTVSS | 36 |
| E007 | EVQLVESGGGLVQPGGSLRLSCEASGFSFSDHTMRWHRQAAGKNLELVAYITPAGD IIKYADSVQGRFTISRDNAKNMLYLQMDSLRPDDTAVYRCNALVSAAVRASDYWG QGTQVTVSS | 37 |
| E008 | EVQVVESGGGLVQSGGSLTLSCTSSGLPLLDYAIGWFRQAPGKEREAVSAISISEGST YYTDSVRGRFTISRDNNKNKVYLRMDNLKPEDTAVYYCAADKTDIGFWLLVGGAG GGEVRYEYWGQGTQVTVSS | 38 |
| E009 | QVQLVESGGGLVQPGGSLRLSCEASGRIDIIFTMGWYRQTPGNEREFVASVTNYNAK YADSVKGRFTISRENTENTAYLQMNSLKPEDTAIYVCHLNYASDYTEYDYYGQGTQ VTVSS | 39 |

### Example 8: Humanized Modification and Affinity Detection of VHH Antibody

Referring to the method of humanizing nano antibodies (J. Biol. Chem. 2009; 284 : 3273-3284), the CDR region transplantation method was used to humanize the four VHH antibodies E002, E003, E005, E006, E008 that bind EGFR antigen. In IgBLAST (http://www.ncbi.nlm.nih.gov/igblast/) Germline with high homology with E002, E003, E005, E006 and E008 was selected as the template. The modified antibody sequences of each antibody are shown in Table 8 below. The effect of the modified VHH antibody on human EGFR was determined by surface plasmon resonance (SPR) Binding kinetics and affinity of his antigen. The results are shown in Table 9. The affinity of the modified antibody is good.

**Table 8: Summary of amino acid sequence of antibody after humanized transformation**

| Antibody number | Amino acid sequence | SEQ ID NO: |
|---|---|---|
| E002.1 | EVQLVESGGGLVQPGGSLRLSCAASGFTFSDYAVSWYRQAPGKEREFVSRITGTGETSRY EDSVKGRFTISRDNSKNTAYLQMNSLRAEDTAVYYCSIYHPPRDYWGQGTQVTVSS | 40 |
| E002.2 | EVQLVESGGGLVQPGGSLRLSCAASGFTFSDYAVSWYRQAPGKEREFVSRITGTGETSRY EDSVKGRFTISRDNVKNTAYLQMNSLRAEDTAVYYCSIYHPPRDYWGQGTQVTVSS | 41 |
| E002.3 | EVQLVESGGGLVQPGGSLRLSCAASGFTFSDYAVSWYRQAPGKEREFVSRITGTGETSRY EDSVKGRFVISRDNSKNTAYLQMNSLRAEDTAVYYCSIYHPPRDYWGQGTQVTVSS | 42 |
| E002.4 | EVQLVESGGGLVQPGGSLRLSCAASGFTFSDYAVSWYRQAPGKEREFVARITGTGETSR YEDSVKGRFTISRDNSKNTAYLQMNSLRAEDTAVYYCSIYHPPRDYWGQGTQVTVSS | 43 |
| E002.5 | EVQLVESGGGLVQPGGSLRLSCAASGFTFSDYAVSWYRQAPGKEREFVARITGTGETSR YEDSVKGRFTISRDNVKNTAYLQMNSLRAEDTAVYYCSIYHPPRDYWGQGTQVTVSS | 44 |
| E002.6 | EVQLVESGGGLVQPGGSLRLSCAASGFTFSDYAVSWYRQAPGKEREFVARITGTGETSR YEDSVKGRFAISRDNSKNTAYLQMNSLRAEDTAVYYCSIYHPPRDYWGQGTQVTVSS | 45 |
| E002.7 | EVQLVESGGGLVQPGGSLRLSCAASGFTFSDYAVSWYRQAPGKEREFVARITGTGETSR YEDSVKGRFAISRDNVKNTAYLQMNSLRAEDTAVYYCSIYHPPRDYWGQGTQVTVSS | 46 |
| E003.1 | QVQLVESGGGLVQPGGSLRLSCSASGGVFSSYAMGWYRQVPGKLRELVATITSGHSTTY ADIVRGRFTISRDNSKNTLYLQMNSLRAEDTAVYYCNRSPYTPGNWGQGTLVTVSS | 47 |
| E003.2 | QVQLVESGGGLVQPGGSLRLSCSASGGVFSSYAMGWYRQVPGKLRELVSTITSGHSTTY ADIVRGRFTISRDNTKNTLYLQMNSLRAEDTAVYYCNRSPYTPGNWGQGTLVTVSS | 48 |
| E003.3 | QVQLVESGGGLVQPGGSLRLSCSASGGVFSSYAMGWYRQVPGKLRELVSTITSGHSTTY ADIVRGRFTISRDNSKNTVYLQMNSLRAEDTAVYYCNRSPYTPGNWGQGTLVTVSS | 49 |
| E003.4 | QVQLVESGGGLVQPGGSLRLSCSASGGVFSSYAMGWYRQVPGKLRELVATITSGHSTTY ADIVRGRFTISRDNTKNTLYLQMNSLRAEDTAVYYCNRSPYTPGNWGQGTLVTVSS | 50 |
| E003.5 | QVQLVESGGGLVQPGGSLRLSCSASGGVFSSYAMGWYRQVPGKLRELVATITSGHSTTY ADIVRGRFTISRDNSKNTVYLQMNSLRAEDTAVYYCNRSPYTPGNWGQGTLVTVSS | 51 |
| E003.6 | QVQLVESGGGLVQPGGSLRLSCSASGGVFSSYAMGWYRQVPGKLRELVATITSGHSTTY ADIVRGRFTISRDNTKNTVYLQMNSLRAEDTAVYYCNRSPYTPGNWGQGTLVTVSS | 52 |
| E003.7 | QVQLVESGGGLVQPGGSLRLSCSASGGVFSSYAMGWYRQVPGKLRELVSTITSGHSTTY ADIVRGRFTISRDNSKNTLYLQMNSLRAEDTAVYYCNRSPYTPGNWGQGTLVTVSS | 53 |
| E003.8 | QVQLVESGGGLVQPGGSLRLSCSASGGVFSSYAMGWYRQVPGKLRELVATITSGHSTTY ADIVRGRFTISRDNSKNTVYLQMNSLEAEDTAVYYCNRSPYTPGNWGQGTLVTVSS | 54 |
| E003.9 | QVQLVESGGGLVQPGGSLRLSCSASGGVFSSYAMGWYRQVPGKLRELVATITSGHSTTY ADIVRGRFTISRDNSKNTVYLQMNRLEAEDTAVYYCNRSPYTPGNWGQGTLVTVSS | 55 |
| E003.10 | QVQLVESGGGLVQPGGSLRLSCSASGGVFSSYAMGWYRQVPGKLRELVSTITSGHSTTY ADIVRGRFTISRDNTKNTVYLQMNRLEAEDTAVYYCNRSPYTPGNWGQGTLVTVSS | 56 |
| E005.1 | QVQLVESGGGLVKPGGSLRLSCAASGFAFSDYTMRWHRQAAGKELELVSYITSAGDIIK YANSVRGRFTISRDNAKNTLYLQMNSLRAEDTAVYRCNALVSASVRASDYWGQGTLVT VSS | 57 |
| E005.2 | QVQLVESGGGLVKPGGSLRLSCAASGFAFSDYTMRWHRQAAGKELELVAYITSAGDIIK YANSVRGRFTISRDNAKNSLYLQMNSLRAEDTAVYRCNALVSASVRASDYWGQGTLVT VSS | 58 |
| E005.3 | QVQLVESGGGLVKPGGSLRLSCAASGFAFSDYTMRWHRQAAGKELELVAYITSAGDIIK YANSVRGRFTISRDNAKNTLYLQMNSLRAEDTAVYRCNALVSASVRASDYWGQGTLVT VSS | 59 |
| E005.4 | QVQLVESGGGLVKPGGSLRLSCAASGFAFSDYTMRWHRQAPGKELELVSYITSAGDIIK YANSVRGRFTISRDNAKNSLYLQMNSLRAEDTAVYRCNALVSASVRASDYWGQGTLVT VSS | 60 |
| E005.5 | QVQLVESGGGLVKPGGSLRLSCAASGFAFSDYTMRWHRQAPGKELELVAYITSAGDIIK YANSVRGRFTISRDNAKNTLYLQMNSLRAEDTAVYRCNALVSASVRASDYWGQGTLVT VSS | 61 |
| E005.6 | QVQLVESGGGLVKPGGSLRLSCAASGFAFSDYTMRWHRQAAGKELELVAYISSAGDIIK YANSVRGRFTISRDNAKNTLYLQMNSLRAEDTAVYRCNALVSASVRASDYWGQGTLVT VSS | 62 |
| E005.7 | QVQLVESGGGLVKPGGSLRLSCAASGFAFSDYTMRWHRQAPGKELELVAYITSAGDIIK YANSVRGRFTISRDNAKNSLYLQMNSLRAEDTAVYRCNALVSASVRASDYWGQGTLVT VSS | 63 |
| E005.8 | QVQLVESGGGLVKPGGSLRLSCEASGFAFSDYTMRWHRQAAGKELELVAYITSAGDIIK YANSVRGRFTISRDNAKNTLYLQMNSLRAEDTAVYRCNALVSASVRASDYWGQGTLVT VSS | 64 |
| E006.1 | EVQLVESGGGLVQPGGSLRLSCAASGFTFDTYTTSWERQAPGKEREMVAVLATTGGKP YYVESVKGRFTISRDNSKNTVYLQMNSLRAEDTAVYYCKATSRSPVNPHNYSGQGTLVT VSS | 65 |
| E006.2 | EVQLVESGGGLVQPGGSLRLSCAASGFTFDTYTTSWERQAPGKEREMVAVLATTGGKP YYVESVKGRFTISRDASKNTVYLQMNSLRAEDTAVYYCKATSRSPVNPHNYSGQGTLVT VSS | 66 |
| E006.3 | EVQLVESGGGLVQPGGSLRLSCAASGFTFDTYTTSWERQAPGKEREMVAVLATTGGKP YYVESVKGRFTISRDASENTVYLQMNSLRAEDTAVYYCKATSRSPVNPHNYSGQGTLVT VSS | 67 |
| E006.4 | EVQLVESGGGLVQPGGSLRLSCAASGFTFDTYTTSWERQAPGKEREMVAVLATTGGKP YYVESVKGRFTISRDASENAVYLQMNSLRAEDTAVYYCKATSRSPVNPHNYSGQGTLVT VSS | 68 |
| E006.5 | EVQLVESGGGLVQPGGSLRLSCAASGFTFDTYTTSWERQAPGKEREMVAVLATTGGKP YYVESVKGRFTISRDASENAVYLQMNNLRAEDTAVYYCKATSRSPVNPHNYSGQGTLV TVSS | 69 |
| E006.6 | EVQLVESGGGLVQPGGSLRLSCAASGFTFDTYTTSWERQAPGKEREMVAVLATTGGKP YYVESVKGRFTISRDASENAVTLQMNNLRAEDTAVYYCKATSRSPVNPHNYSGQGTLV TVSS | 70 |
| E006.7 | EVQLVESGGGLVQPGGSLRLSCAASGFTFDTYTTSWERQAPGKEREMVSVLATTGGKPY YVESVKGRFTISRDASENAVYLQMNSLRAEDTAVYYCKATSRSPVNPHNYSGQGTLVTV SS | 71 |
| E006.8 | EVQLVESGGGLVQPGGSLRLSCTASGFTFDTYTTSWERQAPGKEREMVSVLATTGGKPY YVESVKGRFTISRDASENAVTLQMNSLRAEDTAVYYCKATSRSPVNPHNYSGQGTLVTV SS | 72 |
| E008.1 | EVQLVESGGGLVQPGGSLRLSCAASGLPLLDYAIGWFRQAPGKEREAVSAISISEGSTYY TDSVRGRFTISRDNSKNTVYLQMNSLRAEDTAVYYCAADKTDIGFWLLVGGAGGGEVR YEYWGQGTQVTVSS | 73 |
| E008.2 | EVQLVESGGGLVQPGGSLRLSCAASGLPLLDYAIGWFRQAPGKEREAVSAISISEGSTYY TDSVRGRFTISRDNSKNKLYLQMNSLRAEDTAVYYCAADKTDIGFWLLVGGAGGGEVR YEYWGQGTQVTVSS | 74 |
| E008.3 | EVQLVESGGGLVQPGGSLRLSCAASGLPLLDYAIGWFRQAPGKEREAVSAISISEGSTYY TDSVRGRFTISRDNSKNTVYLRMNSLRAEDTAVYYCAADKTDIGFWLLVGGAGGGEVR YEYWGQGTQVTVSS | 75 |
| E008.4 | EVQLVESGGGLVQPGGSLRLSCAASGLPLLDYAIGWFRQAPGKEREAVSAISISEGSTYY TDSVRGRFTISRDNNKNTVYLQMNSLRAEDTAVYYCAADKTDIGFWLLVGGAGGGEVR YEYWGQGTQVTVSS | 76 |
| E008.5 | EVQLVESGGGLVQPGGSLRLSCAASGLPLLDYAIGWFRQAPGKEREAVSAISISEGSTYY TDSVRGRFTISRDNSKNKVYLQMNSLRAEDTAVYYCAADKTDIGFWLLVGGAGGGEVR YEYWGQGTQVTVSS | 77 |
| E008.6 | EVQLVESGGGLVQPGGSLRLSCAASGLPLLDYAIGWFRQAPGKEREAVSAISISEGSTYY TDSVRGRFTISRDNNKNKVYLQMNSLRAEDTAVYYCAADKTDIGFWLLVGGAGGGEVR YEYWGQGTQVTVSS | 78 |
| E008.7 | EVQLVESGGGLVQPGGSLRLSCASSGLPLLDYAIGWFRQAPGKEREAVSAISISEGSTYYT DSVRGRFTISRDNNKNKVYLQMNSLRAEDTAVYYCAADKTDIGFWLLVGGAGGGEVR YEYWGQGTQVTVSS | 79 |
| E008.8 | EVQLVESGGGLVQPGGSLRLSCSASGLPLLDYAIGWFRQAPGKEREAVSAISISEGSTYYT DSVRGRFTISRDNNKNKVYLQMNSLRAEDTAVYYCAADKTDIGFWLLVGGAGGGEVR YEYWGQGTQVTVSS | 80 |
| E008.9 | EVQLVESGGGLVQPGGSLRLSCAASGLPLLDYAIGWFRQAPGKELEAVSAISISEGSTYY TDSVRGRFTISRDNNKNKVYLQMNSLRAEDTAVYYCAADKTDIGFWLLVGGAGGGEVR YEYWGQGTQVTVSS | 81 |
| E008.10 | EVQLVESGGGLVQPGGSLRLSCASSGLPLLDYAIGWFRQAPGKEREAVSAISISEGSTYYT DSVRGRFTISRDNSKNKVYLRMNSLRAEDTAVYYCAADKTDIGFWLLVGGAGGGEVRY EYWGQGTQVTVSS | 82 |

**Table 9: summary of protein affinity test results**

| Number | ka (1/Ms) | kd (1/s) | KD (M) |
|---|---|---|---|
| E002.1 | 7.8E+04 | 3.9E-04 | 5.0E-09 |
| E002.2 | 1.3E+05 | 6.8E-04 | 5.1E-09 |
| E002.4 | 4.3E+04 | 4.5E-04 | 1.1E-08 |
| E002.5 | 4.3E+04 | 5.5E-04 | 1.3E-08 |
| E002.6 | 4.0E+04 | 3.8E-04 | 9.4E-09 |
| E002.7 | 4.0E+04 | 4.6E-04 | 1.1E-08 |
| E005.1 | 1.1E+04 | 3.5E-05 | 3.3E-09 |
| E005.2 | 1.8E+04 | 2.1E-05 | 1.2E-09 |
| E005.3 | 6.1E+03 | 1.7E-05 | 2.7E-09 |
| E005.4 | 2.0E+03 | 9.6E-06 | 4.8E-09 |
| E005.5 | 1.5E+04 | 1.1E-05 | 7.4E-10 |
| E005.6 | 1.6E+04 | 4.1E-05 | 2.5E-09 |
| E005.7 | 3.5E+03 | 2.5E-06 | 7.1E-10 |
| E005.8 | 1.3E+04 | 2.7E-05 | 2.1E-09 |
| E006.1 | 2.0E+04 | 7.9E-04 | 3.9E-08 |
| E006.2 | 1.7E+04 | 7.2E-04 | 4.1E-08 |
| E006.3 | 2.2E+04 | 4.0E-04 | 1.8E-08 |
| E006.4 | 2.5E+04 | 3.2E-04 | 1.3E-08 |
| E006.5 | 2.4E+04 | 3.1E-04 | 1.3E-08 |
| E006.6 | 2.9E+04 | 3.2E-04 | 1.1E-08 |
| E006.7 | 2.4E+04 | 3.3E-04 | 1.4E-08 |
| E006.8 | 2.7E+04 | 4.0E-04 | 1.5E-08 |
| E008.1 | 4.8E+05 | 8.7E-03 | 1.8E-08 |
| E008.2 | 5.4E+05 | 2.1E-02 | 4.0E-08 |
| E008.3 | 4.9E+06 | 2.8E-02 | 5.8E-09 |
| E008.4 | 4.5E+05 | 9.3E-03 | 2.1E-08 |
| E008.5 | 5.1E+05 | 1.2E-02 | 2.4E-08 |
| E008.6 | 4.3E+05 | 1.3E-02 | 3.2E-08 |
| E008.9 | 4.9E+05 | 1.2E-02 | 2.5E-08 |
| E008.10 | 3.6E+05 | 2.9E-02 | 8.2E-08 |

## Claims

1. A human epidermal growth factor receptor (EGFR ) binding molecule, comprising an anti-EGFR single domain antibody, wherein the complementarity determining regions (CDRs) of the single domain antibody comprise CDR1, CDR2 and CDR3, wherein CDR1 comprises the sequence shown in SEQ ID NO: 1, CDR2 comprises the sequence shown in SEQ ID NO: 2, and CDR3 comprises the sequence shown in SEQ ID NO: 3, wherein,
SEQ ID NO: 1 is GX₁X₂X₃X₄X₅X₆X₇X₈, wherein X₁ is G, X₂ is S, F, G, L or R, X₃ is G, T, V, P, A, S or I, X₄ is F, L or D, X₅ is T, S, E, D, L or I, X₆ is I, D, SorT, X₇ is Q, Y, H or F, and X₈ is A or T;
SEQ ID NO: 2 is X₁X₂X₃X₄X₅X₆X₇X₈, wherein X₁ is I, L or V, X₂ is H, T, F, A or S, X₃ is Q, G, S, P, T, I orN, X₄ is G, T, D, A, S or Y, X₅ is G, H ,E or N, X₆ is S, E, K, D, G or A, X₇ is T, H, I, K, S or null, and X₈ is S, T, I, P or null ;
SEQ ID NO:3 is X₁X₂X₃X₄X₅X₆X₇X₈X₉X₁₀X₁₁X₁₂X₁₃X₁₄X₁₅X₁₆X₁₇X₁₈X₁₉X₂₀X₂₁ X₂₂X₂₃X₂₄X₂₅, wherein X₁ is N, S, A, K or H, X₂ is V, I, R, K, A or L, X₃ is V, Y, S, D, L, T or N, X₄ is P, H, L, V, S, K or Y, X₅ is P, Y, S, R, T or A, X₆ is L, P, T, F, A, S or D, X₇ is R, P, F, S, A, I or D, X₈ is V, D, G, A or Y, X₉ is Y, N, T, R, F or null, X₁₀ is P, A, W, E or null, X₁₁ is S, H, L, Y or null, X₁₂ is F, D, N, L or null, X₁₃ is Y, V or null, X₁₄ is I, G or null, X₁₅ is G or null , X₁₆ is Y, A or null , X₁₇ is G or null, X₁₈ is G or null, X₁₉ is G or null, X₂₀ is E or null, X₂₁ is V or null, X₂₂ is R or null, X₂₃ is Y or null , X₂₄ is E or null, X₂₅ is Y or null _{∘}

2. The human epidermal growth factor receptor binding molecule according to claim 1, wherein CDR1 of the single domain antibody comprises the sequence shown in any one of SEQ ID NOs: 4-12, CDR2 comprises the sequence shown in any one of SEQ ID NOs: 13-21, and CDR3 comprises the sequence shown in any one of SEQ ID NOs: 22-30,
preferably, the single domain antibody comprises CDR1, CDR2 and CDR3 shown in any of the following groups a1 to a9:
| Group | CDR1 | CDR2 | CDR3 |
|---|---|---|---|
| a1 | 4 | 13 | 22 |
| a2 | 5 | 14 | 23 |
| a3 | 6 | 15 | 24 |
| a4 | 7 | 16 | 25 |
| a5 | 8 | 17 | 26 |
| a6 | 9 | 18 | 27 |
| a7 | 10 | 19 | 28 |
| a8 | 11 | 20 | 29 |
| a9 | 12 | 21 | 30 |

3. The human epidermal growth factor receptor binding molecule according to claim 1 or 2, wherein,
the FR region of the single domain antibody comprises the FR region of any VHH selected from SEQ ID NOs: 31-82, and/or
the single domain antibody VHH is as shown in any one of SEQ ID NOs: 31-82, or has at least 80%, 90%, 95%, 96%, 97%, 98%, or 99% identity to amino acid sequence as shown in any one of SEQ ID NOs: 31-82, and/or
the human epidermal growth factor receptor binding molecule is a monovalent or multivalent single domain antibody, a multispecific single domain antibody, a heavy chain antibody or an antigen binding fragment thereof, an antibody or an antigen binding fragment thereof comprising one, two or more of the single domain antibodies.

4. A polynucleotide, wherein the polynucleotide comprises a sequence selected from:
(1) a coding sequence of the human epidermal growth factor receptor binding molecule according to any one of claims 1-3;
(2) a complementary sequence of (1);
(3) a 5-50 bp fragment of any sequence of (1) or (2).

5. A nucleic acid construct, wherein the nucleic acid construct comprises the polynucleotide of claim 4, preferably, the nucleic acid construct is a recombinant vector or expression vector.

6. A Phage comprising the human epidermal growth factor receptor binding molecule according to any one of claims 1-3, preferably, the human epidermal growth factor receptor binding molecule is displayed on the surface of the phage.

7. A host cell, wherein the host cell:
(1) expresses the human epidermal growth factor receptor binding molecule according to any one of claims 1-3; and/or
(2) comprises the polynucleotide according to claim 4; and/or
(3) comprises the nucleic acid construct according to claim 5.

8. A method for producing human epidermal growth factor receptor binding molecule, comprising: culturing the host cell according to claim 7 under conditions suitable for producing the human epidermal growth factor receptor binding molecule, and optionally purifying the human epidermal growth factor receptor binding molecule from the culture.

9. A pharmaceutical composition, comprising the human epidermal growth factor receptor binding molecule according to any one of claims 1-3, the polynucleotide according to claim 4, the nucleic acid construct according to claim 5, the phage according to claim 6 or the host cell according to claim 7, and pharmaceutically acceptable excipients, preferably, the pharmaceutical composition is used for treating cancer.

10. Use of the human epidermal growth factor receptor binding molecule according to any one of claims 1-3 in the preparation of a medicament for the prevention or treatment of a cancer.

11. A kit for detecting human epidermal growth factor receptor, which is used to evaluate the therapeutic effect of a medicament or diagnose cancer, wherein the kit comprises the human epidermal growth factor receptor binding molecule according to any one of claims 1-3, the polynucleotide according to claim 4, the nucleic acid construct according to claim 5, the phage according to claim 6 or the host cell according to claim 7,
preferably, the kit further comprises a reagent for detecting the binding of human epidermal growth factor receptor to a single domain antibody, an antibody, or an antigen binding fragment thereof,
more preferably, the reagent is a reagent that detects the binding by enzyme-linked immunosorbent assay.

12. A non diagnostic method for detecting the presence of human epidermal growth factor receptor in a sample, wherein the method comprises: incubating a human epidermal growth factor receptor binding molecule according to any one of claims 1-3 with the sample, and detecting the binding of human epidermal growth factor receptor to a single domain antibody, antibody, or antigen binding fragment thereof, thereby determining the presence of human epidermal growth factor receptor in the sample.

13. Use of the human epidermal growth factor receptor binding molecule according to any one of claims 1-3 in the preparation of a kit for detecting human epidermal growth factor receptor in samples, evaluating the therapeutic effect of a medicament, or diagnosing a cancer.
